# EUROPEAN PATENT SPECIFICATION

(11) **EP 4 339 278 B1**
(45) Date of publication and mention of the grant of the patent: **14.10.2026**
(21) Application number: 21941743.3
(22) Date of filing: 24.12.2021
(51) Int. Cl.: G01N 35/00, G01N 35/04, G01N 35/10, B01L 9/06

(54) **DNA AND RNA NUCLEIC ACID CO-EXTRACTION AND DETECTION SYSTEM**
DNA- UND RNA-NUKLEINSÄURE-KOEXTRAKTIONS- UND -NACHWEISSYSTEM
SYSTÈME DE DÉTECTION ET DE CO-EXTRACTION D'ACIDES NUCLÉIQUES D'ADN ET D'ARN

(30) Priority: 13.05.2021 CN 202110523722
(43) Date of publication of application: 20.03.2024
(73) Proprietor: Autobio Labtec Instruments Co., Ltd., Zhengzhou, Henan 450016 (CN)
(72) Inventor: WANG, Chao, Zhengzhou, Henan 450016 (CN); LI, Zhenkun, Zhengzhou, Henan 450016 (CN); SUN, Long, Zhengzhou, Henan 450016 (CN); ZHAO, Peng, Zhengzhou, Henan 450016 (CN); LIU, Yaoji, Zhengzhou, Henan 450016 (CN); WEN, Haoran, Zhengzhou, Henan 450016 (CN); GAO, Weiye, Zhengzhou, Henan 450016 (CN); ZHANG, Chaohui, Zhengzhou, Henan 450016 (CN); LIU, Cong, Zhengzhou, Henan 450016 (CN)
(74) Representative: Kolster Oy Ab
(86) International application number: PCT/CN2021/141190
(87) International publication number: WO 2022/237181

(56) References cited:
- WO-A1-2015/072941
- WO-A1-2021/047499
- CN-A- 103 789 198
- CN-A- 106 520 540
- CN-A- 109 337 793
- CN-A- 112 694 970
- CN-A- 113 150 977
- CN-U- 212 504 899
- CN-U- 214 991 615
- US-B2- 10 071 376

## Description

The present application claims priority to Chinese Patent Application No. 202110523722.5, titled "DNA AND RNA NUCLEIC ACID CO-EXTRACTION AND DETECTION SYSTEM ", filed on May 13, 2021 with the China National Intellectual Property Administration.

### FIELD

The present application relates to the technical field of PCR, and in particular to a DNA and/or RNA nucleic acid co-extraction and detection system.

### BACKGROUND

Molecular diagnosis is widely used in clinical diagnosis due to its high sensitivity, high specificity, convenience and rapidness.

A conventional nucleic acid extraction and amplification system mainly adopt a batch sample introduction mode and has few detection items, the processes such as cultivation, magnetic attraction, and transfer of to-be-detected samples are mainly operated manually, and sample supply, reagent strip supply, and discarding consumables during the operation are mainly completed manually, which results in high working intensity and fatigue of a detector, low detection efficiency, and high detection cost.

Each detection process of the conventional nucleic acid extraction and amplification system is required to be completed separately in independent operating spaces, and the detector is required to shuttle in multiple detection rooms to perform detection, which causes a low detection efficiency.

In summary, how to improve the efficiency of nucleic acid extraction and amplification is an urgent problem to be solved by those skilled in the art.

WO2021047499A1 discloses a device for extracting and detecting nucleic acids, comprising one or more sample receiving modules, one or more lysing modules, one or more extraction modules of nucleic acids, one or more amplification modules of nucleic acids, and a detection module. Also provided is an instrument for diagnosing a molecule in vitro comprising the device for extracting and detecting nucleic acids and an automatic control system. Further provided is an in vitro diagnosis method by means of performing extraction and amplification of nucleic acids in a sample using the device for extracting and detecting nucleic acids.

### SUMMARY

The object of the present application is to provide a DNA and/or RNA nucleic acid co-extraction and detection system, which realizes the automation of barcode scanning, sample adding, incubation and extraction, transfer and mixing, and detection during an extraction and amplification process, and effectively improves the efficiency of extraction and amplification of nucleic acid.

In order to achieve the above object, the present application provides the following technical solutions.

A DNA and/or RNA nucleic acid co-extraction and detection system includes a nucleic acid extraction system, a sealing transfer mixing system, a nucleic acid detection system, and a control system, and the nucleic acid extraction system, the sealing transfer mixing system, and the nucleic acid detection system are connected to the control system;
the nucleic acid extraction system includes a basket for loading an extraction reagent strip and a sample tube, a consumable supply module, a kit loading module, a barcode scanning device for automatically inputting barcode information, a magnetic-attraction incubation device and an automatic sample adding device for transferring liquid into a reaction chamber of the extraction reagent strips or a PCR tube and sealing the PCR tube;
the sealing transfer system includes a PCR transfer device and a high-speed mixing device, and the PCR transfer device is configured for grasping the PCR tube and transferring the PCR tube to the high-speed mixing device for high-speed mixing; and
the nucleic acid detection system includes a PCR amplification device and a fluorescence detection device.

In an embodiment, the basket includes a basket cover plate, a basket bottom plate for holding the extraction reagent strip, and a sample holder for holding the sample tube, the sample holder is detachably connected to the basket bottom plate; and
the basket cover plate is rotatably connected to the basket bottom plate to limit a displacement of the extraction reagent strip in a height direction when the basket cover plate contacts the basket bottom plate.

In an embodiment, the barcode scanning device includes a mounting support, a scanning assembly, a pusher assembly for pushing the extraction reagent strip between a scanning position and a standby position, a pusher moving assembly for driving the pusher assembly to move along a length direction of the mounting support and a detection assembly, the detection assembly is configured to determine whether there is an extraction reagent strip and a sample tubes in a current channel, the detection assembly is in signal connection with the pusher moving assembly, and the scanning assembly and the detection assembly are mounted on a mounting base plate of the pusher assembly.

In an embodiment, the magnetic-attraction incubation device includes an incubation module for heating the reaction chamber, a magnetic attraction module for magnetically attracting the reaction chamber, and a driving assembly, both the incubation module and the magnetic attraction module are connected to the driving assembly, the driving assembly is configured to drive the incubation module and the magnetic attraction module alternately approach the reaction chamber.

In an embodiment, the incubation module includes a heating block having a reagent groove, a heating assembly contacting an inner wall of the reagent groove, a heat-dissipation assembly provided at a bottom of the heating block, and a temperature sensor for detecting the temperature of the heating block, the temperature sensor is connected to the heating block.

In an embodiment, the magnetic attraction module includes a magnet and a rotating assembly for driving the magnet to rotate, the rotating assembly is connected to the magnet;
the magnet includes a first magnetic attraction surface and a second magnetic attraction surface, and the first magnetic attraction surface and the second magnetic attraction surface have different areas and are configured to attract magnetic beads in the reaction chamber.

In an embodiment, the PCR transfer device includes a gripper assembly for grasping or releasing the PCR tube, an cover opening assembly for opening or closing the amplification cover of the PCR amplification device, an X-axis assembly for driving the gripper assembly and the cover opening assembly to move along an X-axis direction, a Y-axis assembly for driving the gripper assembly and the cover opening assembly to move along a Y-axis direction, and a Z-axis assembly for driving the gripper assembly and the cover opening assembly to move along a Z-axis direction; and
the gripper assembly and the cover opening assembly are slidably connected to the Z-axis assembly, the Z-axis assembly is slidably connected to the Y-axis assembly, and the Y-axis assembly is slidably connected to the X-axis assembly.

In an embodiment, the gripper assembly includes a gripper base plate, a gripper movable plate parallel to and arranged below the gripper base plate, a gripper for grasping the PCR tube, and a retraction drive assembly for controlling opening or retraction of the gripper, the retraction drive assembly is arranged on the gripper base plate, and a telescopic end of the retraction drive assembly passes through the gripper base plate to be connected to the gripper movable plate, so as to drive the gripper movable plate to rise or descend; and
the gripper bottom plate and the gripper movable plate are connected to the retraction drive assembly, the gripper opens when the gripper movable plate descends, and the gripper retracts when the gripper movable plate rises.

In an embodiment, the PCR amplification device includes an amplification heating block, an amplification heating plate an amplification radiator, an amplification cover for covering the amplification heating block, and an amplification cooling assembly for improving heat dissipation efficiency of the amplification radiator, the amplification heating block has at least two amplification holes for placing the PCR tubes, and the amplification cover, the amplification heating block, the amplification heating plate, and the amplification radiator are sequentially arranged from top to bottom.

In an embodiment, the fluorescence detection device includes a light source assembly for providing a fluorescent light source, a fluorescence detection assembly for quantitative detection, a light source optical fiber, and a fluorescence detection optical fiber, one side of the amplification hole is connected to one end of the light source optical fiber, and the other side of the amplification hole is connected to one end of the fluorescence detection optical fiber; and
the other end of the light source optical fiber is connected to the light source assembly, and the other end of the fluorescence detection optical fiber is connected to the fluorescence detection assembly.

In an embodiment, the automatic sample adding device includes a reagent needle assembly for extraction and injection of a reagent, a sample adding needle assembly for extraction and injection of sample infusion and a gantry assembly, the reagent needle assembly, the sample adding needle assembly and the gantry assembly are connected to the control system; and
the reagent needle assembly and the sample adding needle assembly are respectively arranged on both sides of the gantry assembly, and the gantry assembly is configured to move along the basket, the consumable supply module, and the kit loading module.

In an embodiment, the reagent needle assembly includes a reagent needle, a pricking control assembly, a pipetting control assembly, a reagent needle lifting assembly, and a reagent needle translation assembly, the reagent needle includes an adapter and a reagent pump communicating with the adapter, the reagent pump is connected to the pipetting control assembly, and the adapter is connected to the pricking control assembly for the pricking control assembly to control the adapter to prick or exit a tip or a sealing plug; and
the reagent needle translation assembly is configured to drive the reagent needle to move along a direction perpendicular to a movement direction of the gantry assembly in a horizontal plane, and the reagent needle lifting assembly is configured to drive the reagent needle to move along a vertical direction.

In an embodiment, the sample adding needle assembly includes multiple pipette pumps provided in parallel, a pipette pump lifting assembly for driving the multiple pipette pumps to go up and down, and a plunger lifting assembly for driving plungers of the multiple pipette pumps to slide relative to pump bodies of the multiple pipette pumps, the multiple pipette pumps are installed on the pipette pump lifting assembly, and the plunger lifting assembly is connected to the plungers of the multiple pipette pumps.

When the DNA and/or RNA nucleic acid co-extraction and detection system according to the present application is in operation, firstly, the sample tube containing the sample to be detected and the extraction reagent strip are loaded at the basket, and the barcode information of the sample tube and the extraction reagent strip is input by the barcode scanning device. After the input is completed, the automatic sample adding device punctures a sealing film of the extraction reagent strip and injects the extraction reagent and the sample to be detected to the reaction chamber of the extraction reagent strip. The magnetic-absorption incubation device performs incubation and magnetic absorption on the sample to be detected, to extract the nucleic acid from the sample to be detected. The automatic sample adding device injects an amplification reagent and a purified product after the incubation and magnetic absorption to the PCR tube, and the PCR tube is sealed with the sealing plug. After the sealing, the PCR transfer device grasps the PCR tube and transfers the PCR tube to the high-speed mixing device. After the mixing is completed, the PCR transfer device transfers the PCR tube to the PCR amplification device to complete the PCR amplification process. After the amplification process is completed, the fluorescence detection device performs fluorescence detection on the PCR tube.

Therefore, the DNA and/or RNA nucleic acid co-extraction and detection system according to the present application realizes the automation of barcode scanning, sample addition, incubation and extraction, transfer and mixing, and fluorescence detection during the detection process, and effectively improves the efficiency of the extraction and amplification of the nucleic acid.

### BRIEF DESCRIPTION OF THE DRAWINGS

In order to more clearly illustrate the technical solutions in the embodiments of the present application or in the conventional technology, the following will briefly introduce drawings required in the description of the embodiments or the conventional technology. Apparently, the drawings in the following description are only the embodiments of the present application. For those skilled in the art, other drawings can also be obtained based on the provided drawings without creative efforts.

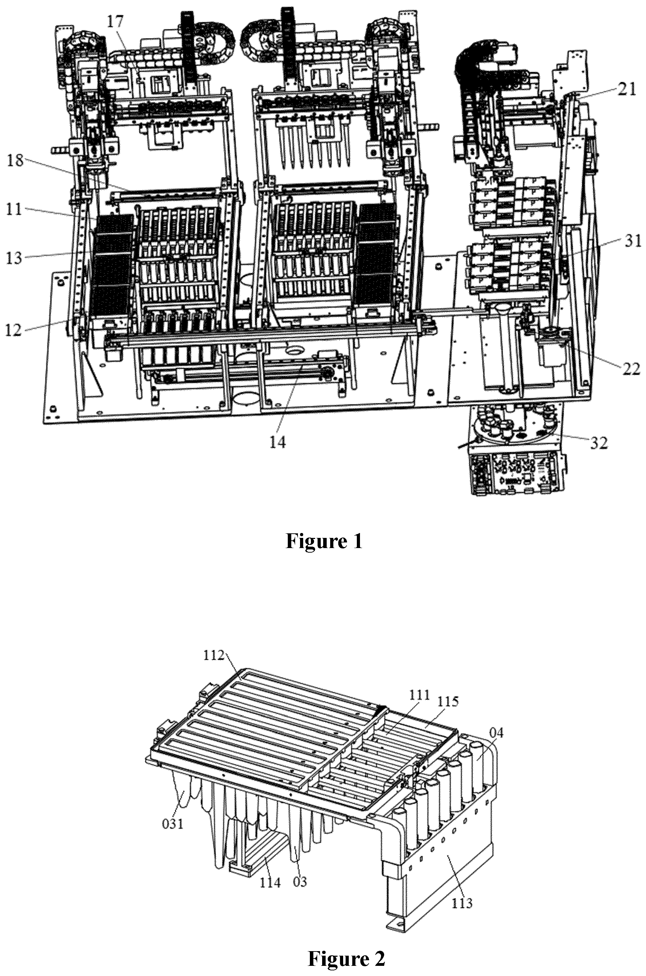

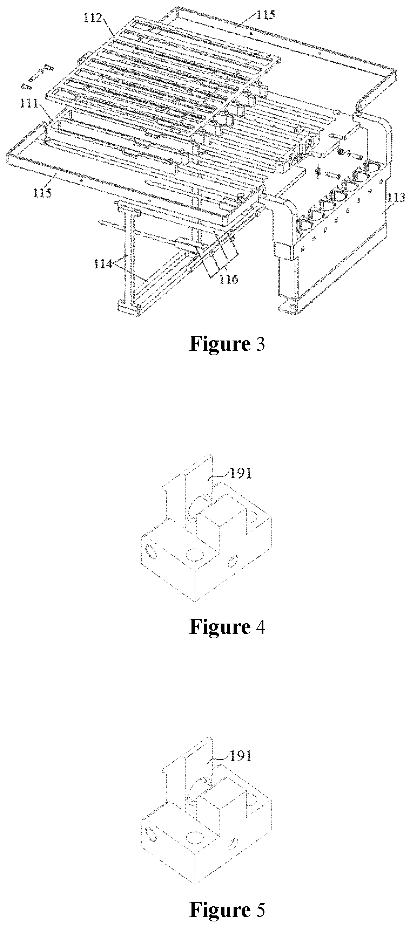

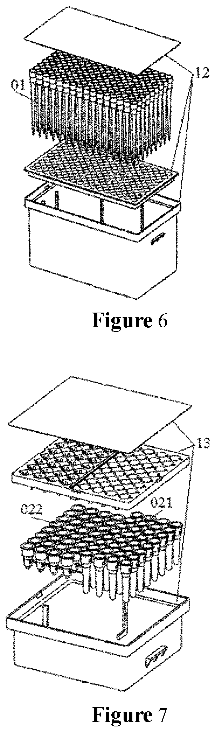

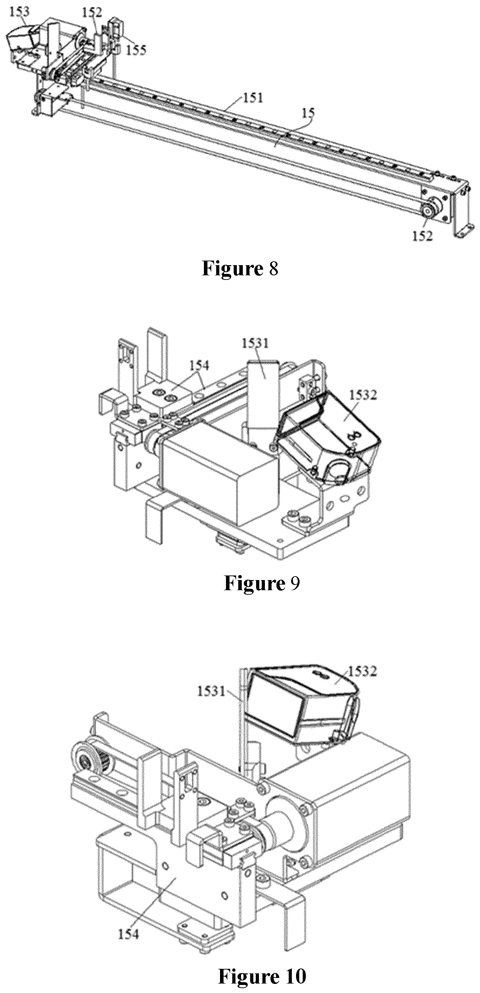

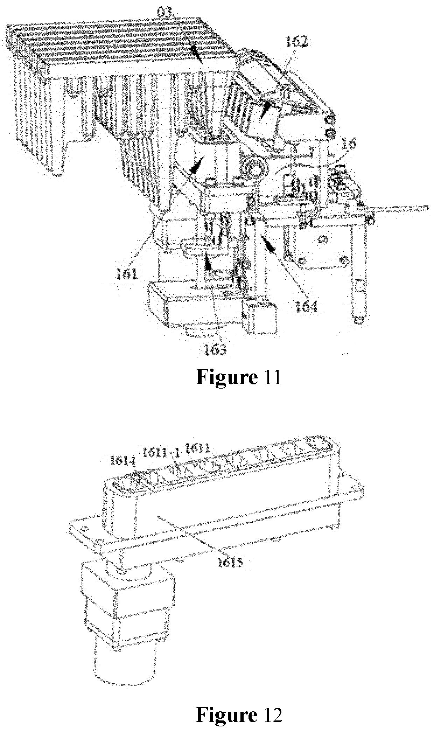

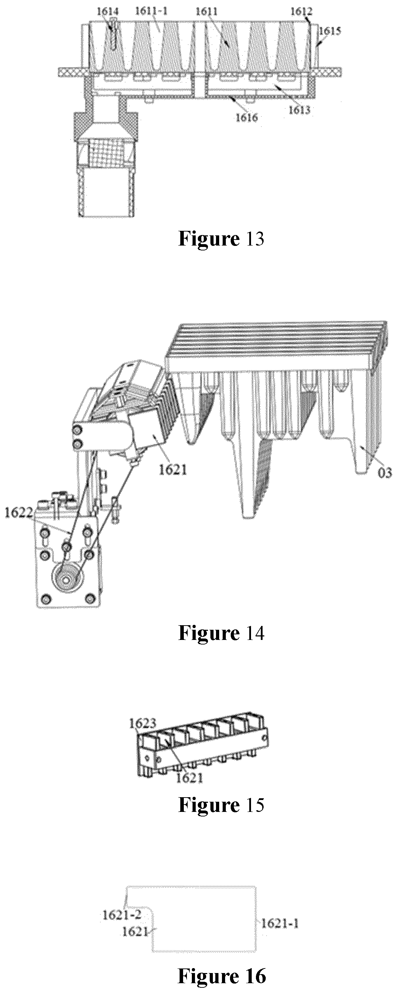

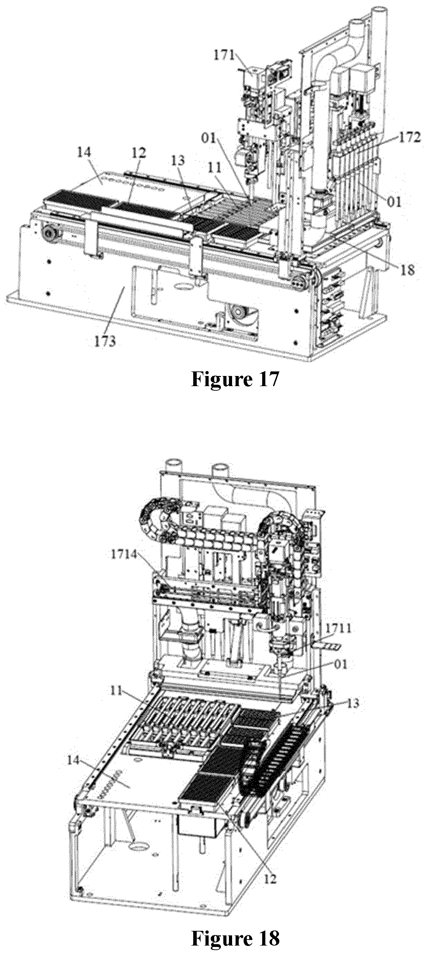

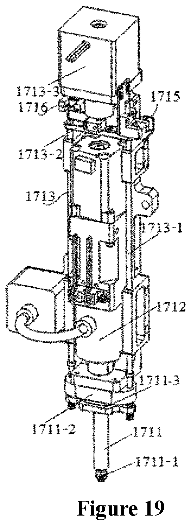

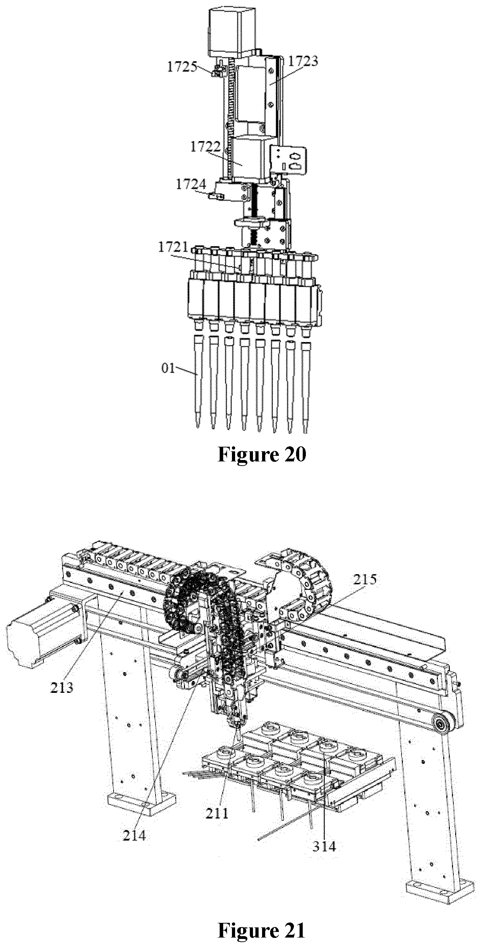

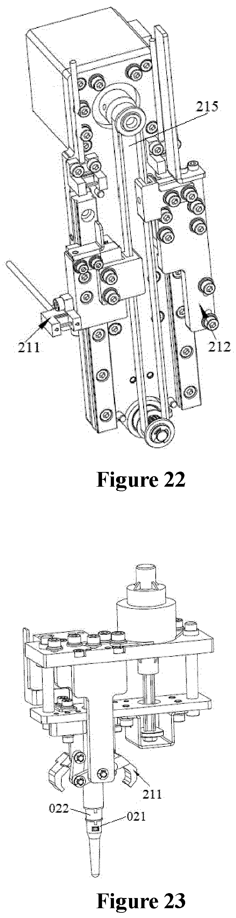

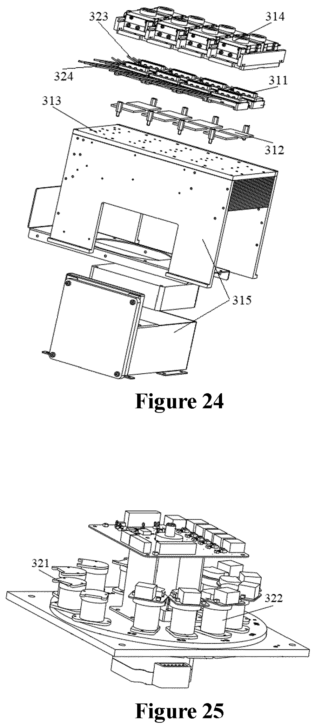

Figure 1 is a schematic structural diagram of a DNA and/or RNA nucleic acid co-extraction and detection system according to an embodiment of the present application; Figure 2 is a schematic structural diagram of a basket; Figure 3 is an exploded schematic diagram of a basket; Figure 4 is a schematic structural diagram of a buckle assembly on a workbench; Figure 5 is a schematic structural diagram of a clamping assembly on a workbench; Figure 6 is a schematic structural diagram of a disposable consumable tip box; Figure 7 is a schematic structural diagram of a PCR consumable box; Figure 8 is a schematic structural diagram of a barcode scanning device; Figure 9 is a schematic structural diagram of a scanning assembly; Figure 10 is a schematic structural diagram of a pusher assembly; Figure 11 is a schematic structural diagram of the magnetic-attraction incubation device; Figure 12 is a schematic structural diagram of an incubation module; Figure 13 is a schematic cross-sectional diagram of Figure 12; Figure 14 is a schematic structural diagram of a magnetic attraction module; Figure 15 is a schematic assembly view of a magnet, a magnet support, and a magnetic isolation plate; Figure 16 is a schematic structural diagram of a magnet; Figure 17 is a schematic structural diagram of an automatic sample adding device; Figure 18 is a schematic structural diagram of an automatic sample adding device in another direction; Figure 19 is a schematic structural diagram of a reagent needle assembly; Figure 20 is a schematic structural diagram of a sample adding needle assembly; Figure 21 is a schematic structural diagram of a PCR transfer device; Figure 22 is a schematic structural diagram of a Z-axis assembly; Figure 23 is a schematic structural diagram of a gripper assembly; Figure 24 is a schematic exploded diagram of a PCR amplification device; and Figure 25 is a schematic structural diagram of a fluorescence detection device.

The reference numbers in Figures 1 to 25 are as follows:
01 is a tip; 021 is a PCR tube, 022 is a sealing plug; 03 is an extraction reagent strip, 031 is a reaction chamber; 04 is a sample tube; 11 is a basket, 111 is a basket bottom plate, 112 is a basket cover plate, 113 is a sample seat, 114 is a support assembly, 115 is a handle, and 116 is a basket positioning assembly; 12 is a disposable tip consumable box; 13 is a PCR consumable box; 14 is a reagent kit loading module; 15 is a barcode scanning device, 151 is a mounting support, 152 is a pusher moving assembly, 153 is a scanning assembly, 1531 is a reflector, 1532 is a code reader, 154 is a pusher assembly, and 155 is a detection assembly; 16 is a magnetic-attraction incubation device, 161 is an incubation module, 162 is a magnetic attraction module, 163 is a driving assembly, 164 is a position-limiting support assembly, 1611 is a heating block, 1611-1 is a reagent groove, 1612 is a heating assembly, 1613 is a heat-dissipation assembly, 1614 is a temperature sensor, 1615 is a heat preservation assembly, 1616 is a cooling assembly, 1621 is a magnet, 1621-1 is a first magnetic attraction surface, 1621-2 is a second magnetic attraction surface, 1622 is a rotating assembly, 1623 is a magnetic isolation plate; 17 is an automatic sample adding device, 171 is a reagent needle assembly, 172 is a sample adding needle assembly, 173 is a gantry assembly, 1711 is an adapter, 1711-1 is a sleeve, 1711-2 is an adapter mounting plate, 1711-3 is a sleeve mounting plate, 1712 is a reagent pump, 1713 is a pricking control assembly, 1713-1 is a guide column, 1713-2 is a guide column connection plate, 1713-3 is a lead-screw motor, 1714 is a reagent needle translation assembly, 1715 is a tip taking detection assembly, 1716 is a tip withdrawing detection assembly, 1721 is a pipette pump, 1722 is a plunger lifting assembly, 1723 is a pipette pump lifting assembly, 1724 is a liquid extraction and injection detection assembly, and 1725 is a Z-axis origin detection assembly; 18 is a liquid-collection and air-extraction device, 191 is a buckle assembly, and 192 is a pressing assembly; 21 is a PCR transfer device, 211 is a gripper assembly, 212 is a cover opening assembly, 213 is an X-axis assembly, 214 is a Y-axis assembly, 215 is a Z-axis assembly, and 22 is a high-speed mixing device; 31 is a PCR amplification device, 311 is an amplification heating block, 312 is a amplification heating plate, 313 is an amplification radiator, 314 is an amplification cover, 315 is an amplification cooling assembly, 32 is a fluorescence detection device, 321 is a light source assembly, 322 is a fluorescence detection assembly, 323 is a light source optical fiber, and 324 is a fluorescence detection optical fiber.

### DETAILED DESCRIPTION OF EMBODIMENTS

The technical solutions in the embodiments of the present application are clearly and completely described below in conjunction with the drawings. Apparently, the described embodiments are only part of the embodiments of the present application, not all of the embodiments of the present application. Any other embodiment obtained by those skilled in the art based on the embodiments in the present application without creative efforts shall fall within the protection scope of the present application.

The core of the present application is to provide a DNA and/or RNA nucleic acid co-extraction and detection system, which realizes the automation of barcode scanning, sample adding, incubation extraction, transfer mixing, and detection during an extraction and amplification process, effectively improves the efficiency of nucleic acid extraction and amplification.

Reference is made to Figures 1 to 25. It should be noted that in the present application, the X-axis direction is a movement direction of a gantry assembly 173 of an automatic sample adding device 17, the Y-axis direction is a direction perpendicular to the X-axis direction in a horizontal plane, and the Z-axis direction is a vertical direction.

A DNA and/or RNA nucleic acid co-extraction and detection system is provided according to the present application, which includes a nucleic acid extraction system, a sealing transfer mixing system, and a nucleic acid detection system. The nucleic acid extraction system includes a basket 11 for loading an extraction reagent strip 03 and a sample tube 04, a consumable supply module, a kit loading module 14, a barcode scanning device 15 for automatically inputting barcode information, a magnetic-attraction incubation device 16, and an automatic sample adding device 17 for transferring liquid into a reaction chamber 031 of the extraction reagent strip 03 or a PCR tube 021 and sealing the PCR tube 021. The sealing transfer mixing system includes a PCR transfer device 21 for grasping the PCR tube 021 and driving the PCR tube 021 to move, and a high-speed mixing device 22. The nucleic acid detection system includes a PCR amplification device 31 and a fluorescence detection device 32.

The consumable supply module is configured to hold disposable consumables required in a detection process, such as a tip 01 and the PCR tube 021. During the detection process, the consumables are taken and placed through the automatic sample adding device 17 and PCR transfer device 21 without manual operation of a detector, which effectively reduces the work intensity of the detector.

Referring to Figure 1, the consumable supply module includes a disposable tip consumable box 12 for holding the tip 01 and a PCR consumable box 13 for holding the PCR tube 021 and a sealing plug 022 corresponding to the PCR tube 021. The structures, materials, sizes, and positions of the disposable tip consumable box 12 and the PCR consumable box 13 are determined based on actual detection requirements.

The kit loading module 14 is configured to place a variety of amplification reagents required for the construction of a PCR system. The structure, material, size, and position of the kit loading module 14 are determined based on actual detection requirements.

Before a sample to be detected is detected, the sample tube 04 containing the sample to be detected and the extraction reagent strip 03 is loaded at the basket 11, and the barcode scanning device 15 is used to input barcode information of the sample tube 04 and the extraction reagent strip 03. During detection, the basket 11 is fixed to a workbench, the automatic sample adding device 17 pricks a sealing film of the extraction reagent strip 03, and extraction reagent and sample solution of the sample to be detected is gradually injected into the reaction chamber 031 of the extraction reagent strip 03. After sample adding is completed, the magnetic-attraction incubation device 16 performs incubation and magnetic attraction treatment on a mixed liquid to extract nucleic acid in the mixed liquid. After the extraction is completed, the automatic sample adding device 17 gradually injects an amplification reagent and a purified product after the incubation and the magnetic attraction into the PCR tube 021, and the PCR tube 021 is sealed with a sealing plug 022 after the pipetting is completed. After the sealing is completed, the PCR transfer device 21 grasps the PCR tube 021 and transfers the PCR tube 021 to the high-speed mixing device 22 for high-speed mixing. After the mixing is completed, the PCR transfer device 21 transfers the PCR tube 021 to the PCR amplification device 31 to perform a PCR amplification process. After the amplification process is completed, the fluorescence detection device 32 performs fluorescence detection on the PCR tube 021.

Therefore, the DNA and/or RNA nucleic acid co-extraction and detection system according to the embodiment realizes the automation of barcode scanning, sample adding, incubation extraction, transfer mixing, and fluorescence detection during the detection process, which effectively improves the efficiency of nucleic acid extraction and amplification.

In an embodiment, the nucleic acid extraction system is also provided with a liquid-collection and air-extraction device 18. The liquid-collection and air-extraction device 18 includes a liquid-collection box for collecting dripping reagents during pipetting and an air-extraction device for performing air-extraction on extracted and injected liquid. The liquid-collection box can effectively prevent cross contamination caused by reagent dripping on an operating surface. The air-extraction device can extract polluted gas generated by extraction and injection of the liquid, avoiding pollution caused by aerosol diffusion.

On the basis of the above embodiments, the basket 11 includes a basket cover plate 112, a basket bottom plate 111 for holding the extraction reagent strip 03, and a sample holder 113 for holding the sample tube 04. The sample holder 113 is detachably connected to the basket bottom plate 111. The basket cover plate 112 is rotatably connected to the basket bottom plate 111 to limit the displacement of the extraction reagent strip 03 in the height direction when the basket cover plate 112 contacts the basket bottom plate 111.

Referring to Figure 2, multiple reagent strip mounting holes arranged in parallel along a length direction of the basket bottom plate 111 are formed in the center of the basket bottom plate 111, and multiple sample tube mounting holes are formed in the sample holder 113. The sizes and quantities of reagent strip mounting holes and sample tube mounting holes are determined based on actual detection requirements.

The basket cover plate 112 is rotatably connected to the basket bottom plate 111 to prevent the extraction reagent strip 03 from moving in a height direction of the basket 11. In an embodiment, the basket cover plate 112 is rotatably connected to one end of the basket bottom plate 111 in the length direction of the basket bottom plate 111. Reagent strip positioning holes in one-to-one correspondence with the reagent strip mounting holes are formed in the basket cover plate 112.

In an embodiment, a first magnet is provided on a top surface of the basket bottom plate 111, and a second magnet is provided on a bottom plate of the basket cover plate 112. When the basket cover plate 112 contacts the basket bottom plate 111, the first magnet is attracted to the second magnet. Therefore, the attraction between the first magnet and the second magnet is to be overcome when the extraction reagent strip 03 is lifted or disengaged, which realizes effective limiting and accurate positioning of the extraction reagent strip 03 in the height direction of the basket 11. In addition, the first magnet and the second magnet may also be replaced with similar positioning structures such as a positioning groove and a positioning block that are engaged with each other.

In order to facilitate the loading of the extraction reagent strip 03 and the sample tube 04, in an embodiment, a support assembly 114 is provided on the bottom surface of the basket bottom plate 111. Specifically, the support assembly 114 may be an H-shaped structure as shown in Figure 2, or may be support pillars provided at four corners of the basket bottom plate 111, or may be other conventional support structures.

When the extraction reagent strip 03 and the sample tube 04 are being loaded, the basket cover plate 112 is rotated, the extraction reagent strip 03 and the sample tube 04 are respectively placed in the basket bottom plate 111 and the sample holder 113, and the basket cover plate 112 is rotated after placement is completed, so that the basket bottom plate 111 contacts the basket cover plate 112. After the loading is completed, the basket 11 is placed on the workbench and is fixed. After the detection is completed, the basket 11 is removed from the workbench, and the extraction reagent strip 03 and the sample tube 04, that are used, are discarded.

In the embodiment, batch synchronous loading of the extraction reagent strips 03 and the sample tubes 04 is realized, which saves the tedious process of multiple placements, and the placement efficiency is high. The basket 11 can accurately locate the extraction reagent strip 03 and the sample tube 04 that are placed in the basket 11, so as to avoid repositioning the extraction reagent strips 03 and the sample tubes 04 after the basket 11 is placed on the workbench.

In order to fix the basket 11, a buckle assembly 191 and a pressing assembly 192 are provided on the workbench for positioning the basket 11. The buckle assembly 191 and the pressing assembly 192 are respectively engaged with two ends of the basket 11 in the length direction. The structures, sizes, materials, quantities, and set positions of the buckle assembly 191 and the pressing assembly 192 are determined according to actual production requirements, which are not described here.

In an embodiment, in order to facilitate the transfer of the basket 11 and improve transfer efficiency, the basket bottom plate 111 is rotatably connected to a handle 115. The structure, shape, size, quantity, material, and position of the handle 115 are determined according to actual requirements.

In an embodiment, the basket 11 further includes a basket positioning assembly 116. The basket positioning assembly 116 includes a guide seat, an extension spring, a guide shaft provided along the length direction of the basket bottom plate 111, and a basket baffle for limiting the extraction reagent strip 03. The guide seat is connected to the bottom surface of the basket bottom plate 111. The guide shaft is slidably connected to the guide seat, one end of the guide shaft is vertically connected to the basket baffle, and two ends of the extension spring are respectively connected to the basket baffle and the bottom surface of the basket bottom plate 111.

Referring to Figure 3, one end, close to the basket bottom plate 111 and the basket cover plate 112, of a rotating shaft is a front end of the basket bottom plate 111. When the extraction reagent strip 03 is being loaded, the extension spring is manually pulled to drive the basket baffle to move towards the front end of the basket bottom plate 111. The basket baffle is located on a right side of the extraction reagent strip 03, so the basket baffle pushes the extraction reagent strip 03 to move towards the front end of the basket bottom plate 111 until the extraction reagent strip 03 contacts a left wall surface of the reagent strip mounting hole. At this time, placement positions of the extraction reagent strips 03 are same.

On the basis of the above embodiments, the barcode scanning device 15 includes a mounting support 151, a scanning assembly 153, a pusher assembly 154 for pushing the extraction reagent strip 03 between a scanning position and a standby position, a pusher moving assembly 152 for driving the pusher assembly 154 to move along a length direction of the mounting support 151, and a detection assembly 155. The detection assembly 155 is configured to determine whether there is an extraction reagent strip 03 and a sample tube 04 in a current channel. The detection assembly 155 is in signal connection with the pusher moving assembly 152. The scanning assembly 153 and the detection assembly 155 are mounted on a mounting base plate of the pusher assembly 154.

The pusher moving assembly 152 includes a linear displacement mechanism and a driving mechanism. The linear displacement mechanism is arranged along the length direction of the mounting support 151, and specifically, is a combination of a conventional linear displacement structure such as a synchronous belt mechanism and a guide-rail and slider mechanism, and a driving mechanism such as a step motor, an electric push rod, and a hydraulic cylinder.

Referring to Figure 9, the scanning assembly 153 includes a reflector 1531 and a code reader 1532. The reflector 1531 is configured to reflect detection light of the code reader 1532 to a barcode of the sample tube 04, so that the code reader 1532 performs code-scanning input on the sample tube 04. The type and size of the code reader 1532, the type and size of the reflector 1531, and a relative position relation between the reflector 1531 and code reader 1532 can be determined with reference to the prior art and according to actual requirements, which are not described here.

The pusher assembly 154 includes a linear displacement mechanism, a driving mechanism, and a pusher that pushes the extraction reagent strip 03 to move. The pusher is connected to the linear displacement mechanism. The specific configuration solutions of the linear displacement mechanism and the driving mechanism refer to the pusher moving assembly 152.

In an embodiment, referring to Figure 10, the mounting base plate of the pusher assembly 154 is provided with a push block for blocking the extraction reagent strip 03, a line rail, a synchronous belt mechanism, and a step motor for driving the synchronous belt mechanism. The push block is slidably connected to the line rail, and a length direction of the line rail is perpendicular to a length direction of the mounting support 151. When the step motor rotates, the synchronous belt mechanism is driven to drive the push block to slide relative to the line rail, and the push block contacts the extraction reagent strip 03, so that the extraction reagent strip 03 moves between the standby position and the scanning position.

The detection assembly 155 includes a reagent strip detection sensor for detecting whether there is an extraction reagent strip 03 in the current channel and a sample tube detection sensor for detecting whether there is a sample tube 04 in the current channel. The reagent strip detection sensor is in signal connection with the pusher assembly 154.

The pusher assembly 154 drives the extraction reagent strip 03 to move from the standby position to the scanning position when the reagent strip detection sensor detects the presence of the extraction reagent strip 03. The scanning assembly 153 scans barcode information of the sample tube 04 when the sample tube detection sensor detects the presence of the sample tube 04. When neither of the sensors detects barcode information, the pusher moving assembly 152 drives the pusher assembly 154 to move to a next channel.

During operation, the detection assembly 155 first determines whether there are an extraction reagent strip 03 and a sample tube 04 in the current channel. If yes, the pusher assembly 154 drives the extraction reagent strip 03 to move to the scanning position, and the scanning assembly 153 scans two-dimensional codes of the extraction reagent strip 03 and/or the sample tube 04. After the scanning is completed, the pusher assembly 154 drives the extraction reagent strip 03 to return to the standby position, and the pusher moving assembly 152 drives the pusher assembly 154 and the scanning assembly 153 to move to the next channel. If not, the pusher moving assembly 152 directly drives the pusher assembly 154 and the scanning assembly 153 to move to the next channel.

In the embodiment, the scanning assembly 153 and the pusher assembly 154 can move along the length direction of the mounting support 151, and can read barcode information of multiple channels. The scanning assembly 153 can scan the extraction reagent strip 03 and the sample tube 04, and the detection speed is fast. Meanwhile, the barcode scanning device 15 has a compact structure and can operate in a narrow and long space, which is beneficial for reducing the size of the DNA and/or RNA nucleic acid co-extraction and detection system and improves space utilization.

In an embodiment, the detection assembly 155 further includes a pusher in-place sensor for detecting whether the extraction reagent strip 03 reaches the scanning position and a pusher reset sensor for detecting whether the extraction reagent strip 03 returns to the standby position. The pusher in-place sensor and the pusher reset sensor are both in signal connection with the pusher assembly 154. Both the pusher in-place sensor and the pusher in-place sensor can be configured as proximity switches, position limit switches, etc.

When the pusher assembly 154 moves to a last channel of the mounting support 151, the pusher moving assembly 152 can be controlled to drive the pusher assembly 154 to reset, and the extraction reagent strip 03 and the sample tube 04 in each channel can be detected sequentially from a first channel. The pusher moving assembly 152 also may be controlled to drive the scanning assembly 153 to perform reverse scanning on a next set of the extraction reagent strip 03 and the sample tube 04 to be detected.

In an embodiment, in order to achieve the reset of the scanning assembly 153, the detection assembly 155 may further include a scanning reset sensor for detecting whether the scanning assembly 153 moves to an end of the mounting support 151, and the scanning reset sensor is in signal connection with the pusher moving assembly 152.

On the basis of the above embodiments, the magnetic-attraction incubation device 16 includes an incubation module 161 for heating the reaction chamber 031, a magnetic attraction module 162 for magnetically attracting the reaction chamber 031, and a driving assembly 163. Both the incubation module 161 and the magnetic attraction module 162 are connected to the driving assembly 163. The driving assembly 163 is configured to drive the incubation module 161 and the magnetic attraction module 162 to alternately approach the reaction chamber 031.

After the automatic sample adding device 17 adds extraction reagents and the sample solution of the sample to be detected to the reaction chamber 031, the driving assembly 163 is controlled to drive the incubation module 161 to approach the reaction chamber 031, so as to effectively heat the mixed liquid in the reaction chamber 031 and facilitate the incubation operation of the mixed liquid. At the same time, the magnetic attraction module 162 moves away from the reaction chamber 031 to prevent the influence on the incubation process. After the incubation process is completed, the driving assembly 163 is controlled to drive the magnetic attraction module 162 to approach the reaction chamber 031, so that magnetic beads in the mixed liquid are attracted onto an inner wall surface of the reaction chamber 031, and at the same time, the incubation module 161 moves away from the reaction chamber 031. After the magnetic attraction operation is completed, the driving assembly 163 is controlled to drive the magnetic attraction module 162 to move away from the reaction chamber 031.

In the embodiment, the driving assembly 163 is configured to alternately control the incubation module 161 and the magnetic attraction module 162 to approach the reaction chamber 031, thereby realizing contact and separation between the incubation module 161 and the reaction chamber 031, so as to prevent slow temperature increase and decrease of the mixed liquid caused by the continuous heating of the mixed liquid in the reaction chamber 031 by the incubation module 161, and improve the operating efficiency of the device. At the same time, the integrated configuration of the incubation module 161 and the magnetic attraction module 162 effectively reduces the overall size of the device and simplifies operation steps of the incubation and the magnetic attraction.

The driving assembly 163 may be a variety of driving mechanisms such as a lead-screw driving mechanism and a guide rail driving mechanism. In an embodiment, the driving assembly 163 is a lead-screw driving mechanism. The lead-screw driving mechanism may be vertically arranged as shown in Figure 11, or horizontally arranged according to the overall layout of the magnetic-attraction incubation device 16, as long as the driving assembly 163 can drive the incubation module 161 to rise and fall.

In an embodiment, the magnetic-attraction incubation device 16 may further include a position-limiting support assembly 164. Both the magnetic attraction module 162 and the driving assembly 163 are connected to the position-limiting support assembly 164. the position-limiting support assembly 164 is provided with a position-limiting rail for limiting a lateral movement trajectory of the magnetic attraction module 162. The magnetic attraction module 162 moves away from the reaction chamber 031 along the position-limiting rail when the driving assembly 163 drives the incubation module 161 to rise. When the driving assembly 163 drives the incubation module 161 to descend, the magnetic attraction module 162 approaches the reaction chamber 031 along the position-limiting rail.

Referring to Figures 11 to 14, in an embodiment, the position-limiting support assembly 164 includes a horizontal support plate and a vertical support plate. The horizontal support plate and the vertical support plate are vertically connected. The position-limiting rail is arranged on the horizontal support plate, and the magnetic attraction module 162 is slidably connected to the position-limiting rail. A synchronous belt mechanism connected to the magnetic attraction module 162 is provided on a top of the vertical support plate along a vertical direction. A blocking piece is provided on the synchronous belt mechanism, and a power piece for abutting against the blocking piece is provided at a telescopic end of the driving assembly 163.

When the telescopic end of the driving assembly 163 drives the incubation module 161 to descend, the power piece abuts against the blocking piece, so that the power piece can drive the blocking piece to move downwards, and a synchronous belt of the synchronous belt mechanism moves downwards and is tensioned, thereby allowing the magnetic attraction module 162 to approach the vertical support plate and the reaction chamber 031 along the position-limiting rail. On the contrary, the power piece moves upwards and separates from the blocking piece when the telescopic end of the driving assembly 163 drives the incubation module 161 to rise, which drives the magnetic attraction module 162 to move away from the reaction chamber 031. Therefore, the lifting movement of the incubation module 161 and the movement of the magnetic attraction module 162 approaching or moving away from the reaction chamber 031 are controlled by the same driving assembly 163, simplifying the overall structure of the magnetic-attraction incubation device 16.

In order to facilitate the reset of the magnetic attraction module 162, a magnetic-attraction reset spring may be provided on the horizontal support plate, and two ends of the magnetic-attraction reset spring are respectively connected to the vertical support plate and the magnetic attraction module 162. Alternatively, a magnetic-attraction moving assembly may be provided to drive the magnetic attraction module 162 to move along a position-limiting assembly. The magnetic attraction module 162 is installed on the magnetic-attraction moving assembly. The magnetic-attraction moving assembly may specifically include a linear displacement mechanism such as a linear guide rail mechanism and a driving mechanism such as a lead-screw motor and a hydraulic cylinder.

On the basis of the above embodiments, the incubation module 161 includes a heating block 1611 having a reagent groove 1611-1, a heating assembly 1612 contacting an inner wall surface of the reagent groove 1611-1, a heat-dissipation assembly 1613 provided at a bottom of the heating block 1611, and a temperature sensor 1614 for detecting the temperature of the heating block 1611. The temperature sensor 1614 is connected to the heating block 1611.

In order to realize simultaneous incubation of multiple reaction chambers 031, multiple reagent grooves 1611-1-1 are formed in the heating block 1611, so that incubation temperatures of the multiple reaction chambers 031 are the same during heating, solving the problem of temperature uniformity, improving the accuracy of detection results and reducing experimental errors. The specific number of the reagent grooves 1611-1 is determined based on actual requirements and is not repeated here.

In an embodiment, the heating power of the heating assembly 1612 is distributed in a non-uniform distribution on the inner wall surfaces of the reagent grooves 1611-1, so as to ensure temperature uniformity at various positions inside the heating block 1611.

In an embodiment, a heat preservation assembly 1615 is provided on a periphery of the heating block 1611, and a cooling assembly 1616 is provided below the heat-dissipation assembly 1613 to improve heat dissipation efficiency and facilitate rapid switching of incubation temperatures within the incubation temperature range.

The structures, materials, sizes, positions, and connection manners of the heating block 1611, the heating assembly 1612, the heat-dissipation assembly 1613, the temperature sensor 1614, the heat preservation assembly 1615, and the cooling assembly 1616 are determined by reference to the conventional technology according to the actual incubation requirements, and are not described here.

When the incubation module 161 is working, the heating assembly 1612 first heats the heating block 1611. When the temperature sensor 1614 detects that the temperature of the heating block 1611 reaches a first preset temperature (usually set to 37 °C), the heating operation of the heating assembly 1612 is stopped, and the heat preservation assembly 1615 maintains the temperature of the heating block 1611 constant, so as to perform incubation on the sample solution in the reaction chamber 031.

After a first incubation is completed and magnetic attraction is completed, the heating assembly 1612 is used again to quickly heat the heating block 1611. When the temperature sensor 1614 detects that the temperature of the heating block 1611 reaches a second preset temperature (usually set to 80 °C), the heating operation of the heating assembly 1612 is stopped, and the heat preservation assembly 1615 is activated to maintain the temperature of the heating block 1611 constant, so as to incubating the sample solution again.

After the incubation of the sample solution is completed, the heat-dissipation assembly 1613 is used to cool the heating block 1611, and at the same time, the cooling assembly 1616 is activated to improve the heat dissipation efficiency of the heat-dissipation assembly 1613.

In the embodiment, the heating assembly 1612 contacts the inner wall surface of the reagent groove 1611-1, so that the temperature of the reagent groove 1611-1 increases rapidly. The cooling assembly 1616 improves the heat dissipation efficiency of the heat-dissipation assembly 1613, so that the temperature of the reagent groove 1611-1 decreases rapidly, thereby achieving a rapid transformation of the incubation temperature and improving the incubation efficiency.

On the basis of the above embodiments, the magnetic attraction module 162 includes a magnet 1621 and a rotating assembly 1622 for driving the magnet 1621 to rotate. The rotating assembly 1622 is connected to the magnet 1621. The magnet 1621 has a first magnetic attraction surface 1621-1 and a second magnetic attraction surface 1621-2. The first magnetic attraction surface 1621-1 and the second magnetic attraction surface 1621-2 have different areas and are configured to attract magnetic beads in the reaction chamber 031.

Multiple magnets 1621 are arranged in parallel with each other, and the number of the magnets 1621 is the same as the number of the reagent grooves 1611-1 in the incubation module 161. The areas of the first magnetic attraction surface 1621-1 and the second magnetic attraction surface 1621-2 are different, so as to change the magnetic force of the magnetic attraction module 162 by switching between the first magnetic attraction surface 1621-1 and the second magnetic attraction surface 1621-2.

Referring to Figures 17 to 18, an area of the first magnetic attraction surface 1621-1 is greater than an area of the second magnetic attraction surface 1621-2. Therefore, when the reaction chamber 031 requires a larger magnetic force, the rotating assembly 1622 is controlled to rotate the first magnetic attraction surface 1621-1 to face a mating surface of the reaction chamber 031. On the contrary, when a small magnetic force is required, the rotating assembly 1622 is controlled to rotate the second magnetic attraction surface 1621-2 to face the mating surface of the reaction chamber 031.

When the magnetic attraction module 162 is working, the magnetic attraction surface facing the reaction chamber 031 is determined based on the required magnetic force, and the rotating assembly 1622 is controlled to make the magnetic attraction surface face the mating surface of the reaction chamber 031. When it is necessary to change the magnetic attraction surface, the magnetic attraction module 162 is controlled to move away from the reaction chamber 031, and then the rotating assembly 1622 is controlled to rotate to allow the other magnetic attraction surface to face the reaction chamber 031. After the adjustment is completed, the magnetic attraction module 162 is controlled to approach the reaction chamber 031 and the other magnetic attraction surface is allowed to contact the mating surface of the reaction chamber 031.

In the embodiment, the rotating assembly 1622 realizes magnetic force change through the conversion of the magnetic attraction surfaces. The structure is simple, the operation is convenient, and the popularization is convenient.

In an embodiment, in order to avoid the interference of the magnet 1621 on the magnetic bead suspension in the reaction chamber 031 in a non-magnetic-attraction state, the magnetic attraction module 162 is provided with a magnetic isolation plate 1623 for magnetism isolation of the magnetic beads during a non-magnetic-attraction process. The shape, size, material, connection manner, connection position, etc. of the magnetic isolation plate 1623 are determined based on actual detection requirements, which are not described here.

In an embodiment, the rotating assembly 1622 includes a synchronous belt mechanism for driving a rotating shaft of the magnetic attraction module 162 and a rotating motor for driving the synchronous belt mechanism. The shapes, structures, sizes, materials, positions, etc. of the rotating motor, the synchronous belt mechanism, and the rotating shaft are determined based on the actual detection requirements, which are not described here. When the rotating motor works, the rotating motor drives the synchronous belt mechanism to rotate, thereby driving the rotating shaft of the magnetic attraction module 162 to rotate. When the rotating shaft rotates, the magnet 1621 rotates circumferentially to switch between the first magnetic attraction surface 1621-1, the second magnetic attraction surface 1621-2, and the magnetic isolation plate 1623, thereby realizing magnetic switching and magnetic isolation operation.

Alternatively, the rotating shaft may also be directly driven by the rotating motor.

On the basis of the above embodiments, the automatic sample adding device 17 includes a reagent needle assembly 171 for extraction and injection of the reagent, a sample adding needle assembly 172 for injection and extraction of the sample solution, and a gantry assembly 173. The reagent needle assembly 171, the sample adding needle assembly 172, and the gantry assembly 173 are connected to a control system. The reagent needle assembly 171 and the sample adding needle assembly 172 are respectively arranged on both sides of the gantry assembly 173. The gantry assembly 173 can move along the basket 11, the consumable supply module, and the kit loading module 14.

The reagent needle assembly 171 is responsible for the extraction and injection of the reagent, the sample adding needle assembly 172 is responsible for addition and mixing of the sample solution, and the gantry assembly 173 is responsible for transferring and positioning the reagent needle assembly 171 and the sample adding needle assembly 172, so as to realize multi-channel synchronous operation and improve detection efficiency of equipment.

Referring to Figure 17, the basket 11, the consumable supply module, and the kit loading module 14 are arranged side by side on the workbench at an upper end of the gantry assembly 173.

When the automatic sample adding device 17 is working, firstly, the control system controls the gantry assembly 173 to drive the sample adding needle assembly 172 to move to an area where the basket 11 loads the extraction reagent strip 03. The sample adding needle assembly 172 pricks and takes the tip 01 and pricks an aluminum plastic film on a top of the extraction reagent strip 03 one by one at each hole position. The gantry assembly 173 drives the reagent needle assembly 171 to move to the position above the disposable tip consumable box 12, and the reagent needle assembly 171 pricks and takes the tip 01. The gantry assembly 173 drives the reagent needle assembly 171 to move to the position above the kit loading module 14, and the reagent needle assembly 171 extracts the reagent. The gantry assembly 173 drives the reagent needle assembly 171 to move to the position above the extraction reagent strip 03, and the reagent needle assembly 171 adds the extraction reagent to the reaction chamber 031 of the extraction reagent strip 03. After the adding is completed, the gantry assembly 173 drives the reagent needle assembly 171 to move to the position above a discarding hole position, and the reagent needle assembly 171 exits the tip 01 and discards the tip 01. The above steps are repeated to complete the sample adding process of all the extraction reagents. The gantry assembly 173 drives the sample adding needle assembly 172 to extract the sample solution in the sample tube 04 loaded in the basket 11 and add the sample solution to the reaction chamber 031 of the extraction reagent strip 03, and perform multiple extraction and injection operations on the mixed solution in the reaction chamber 031 to fully mix the extraction reagent and the sample solution. After the mixing is completed, the gantry assembly 173 drives the sample adding needle assembly 172 to move away from the extraction reagent strip 03 to facilitate the subsequent incubation and magnetic absorption process.

During the magnetic attraction process, the control system controls the gantry assembly 173 to drive the sample adding needle assembly 172 to extract all liquid in the reaction chamber 031 and transfer liquid to a reagent hole of the extraction reagent strip 03. The gantry assembly 173 drives the sample adding needle assembly 172 to extract a washing solution from the extraction reagent strip 03 and inject the washing solution into the reaction chamber 031. At this time, the magnetic attraction module 162 is far away from the reaction chamber 031. During the injection process, the washing solution flushes the magnetic beads condensed on the inner wall of the reaction chamber 031. After the injection is completed, the sample adding needle assembly 172 performs multiple extraction and injection operations to fully mix the magnetic beads and the washing solution.

During the magnetic attraction and incubation process, the reagent needle assembly 171 is at a free period. In order to accelerate the detection and reduce the detection time, preferably, the reagent needle assembly 171 is used to construct the PCR system. Firstly, the gantry assembly 173 drives the reagent needle assembly 171 to move to the position above the kit loading module 14, extracts the amplification reagent and injects the amplification reagent to the PCR tube 021 located in the PCR consumable box 13, and discards the tip 01 after the injection is completed. The above steps are repeated to complete the injection process of all amplification reagents.

After the magnetic attraction and incubation process is completed, the magnetic attraction module 162 remains stationary and still contacts the reaction chamber 031. The control system controls the gantry assembly 173 to drive the reagent needle assembly 171 to move to the position above the area where the basket 11 loads the extraction reagent strip 03. A purified product in the reaction chamber 031 is extracted and injected to the PCR tube 021 containing the amplification reagent. Then, the gantry assembly 173 drives the reagent needle assembly 171 to suck a sealing liquid in the reagent strip 03 and inject the sealing liquid to the PCR tube 021. Finally, the gantry assembly 173 drives the reagent needle assembly 171 to move to the position above the PCR consumable box 13, take the sealing plug 022, and then move over the PCR tube 021, and the sealing plug 022 is used to seal the PCR tube 021.

It should be noted that, during the above process, when the type of the liquid sucked or injected by the reagent needle assembly 171 and the sample adding needle assembly 172 changes, the tip 01 used previously is required to be discarded and replaced to avoid reagent contamination during the pipetting process.

In the embodiment, fully automatic extraction and addition of the sample solution and the reagent are realized, which effectively reduces the labor intensity of the detector and improving the detection efficiency and the operation accuracy.

In an embodiment, the gantry assembly 173 includes a gantry and a sample adding moving assembly that drives the gantry to move. The workbench is provided with a sample adding guide assembly that restricts the movement direction of the gantry. The sample adding guide assembly is slidably connected to the gantry, and the sample adding moving assembly is connected to the control system.

Referring to Figure 17, the reagent needle assembly 171 and the sample adding needle assembly 172 are respectively arranged on both sides of the gantry. When the sample adding moving assembly drives the gantry to move along an extension direction of the sample adding guide assembly, the gantry drives the reagent needle assembly 171 and the sample adding needle assembly 172 to move along the basket 11, the consumable supply module, and the kit loading module 14.

The sample adding guide assembly may be a conventional linear displacement mechanism such as a guide-rail and slider mechanism, a guide-sleeve and guide-column mechanism, or a gear rack mechanism. The sample adding moving assembly may be selected as a driving mechanism such as a driving motor, a hydraulic cylinder, or an electric push rod. The structures, sizes, positions and the like of the sample adding guide assembly and the sample adding moving assembly are determined by reference to the conventional technology according to the actual detection requirements, which are not repeated here.

On the basis of the above embodiments, the reagent needle assembly 171 includes a reagent needle, a pricking control assembly 1713, a pipetting control assembly, a reagent needle lifting assembly, and a reagent needle translation assembly 1714. The reagent needle includes an adapter 1711 and a reagent pump 1712 communicating with the adapter 1711. The reagent pump 1712 is connected to the pipetting control assembly, and the adapter 1711 is connected to the pricking control assembly 1713, so that the pricking control assembly 1713 can control the adapter 1711 to prick or exit the tip 01 or the PCR tube 021. The reagent needle translation assembly 1714 is configured to drive the reagent needle to move along a direction perpendicular to a movement direction of the gantry assembly 173 in a horizontal plane, and the reagent needle lifting assembly is configured to drive the reagent needle to move along the vertical direction.

Referring to Figures 18 and 19, a reagent needle mounting seat is provided in the reagent needle assembly 171. The reagent needle mounting seat is configured to mount the reagent needle, the pricking control assembly 1713, the pipetting control assembly, and the reagent needle lifting assembly. The reagent needle mounting seat is slidably connected to the gantry assembly 173 through the reagent needle translation assembly 1714.

The adapter 1711 is configured to cooperate with the tip 01 or the sealing plug 022. The reagent pump 1712 is a liquid suction pump for connecting and communicating with the adapter 1711, and is connected to a mobile control assembly. The type and size of the adapter 1711, and the type, size, and position of the reagent pump 1712, are determined by reference to the conventional technology according to the actual detection requirements, which are not repeated here.

A sleeve 1711-1 is sleeved on the adapter 1711 for protecting the adapter 1711. The adapter 1711 and the sleeve 1711-1 are respectively mounted on an adapter mounting plate 1711-2 and a sleeve mounting plate 1711-3. An elastic member is provided between the adapter mounting plate 1711-2 and the sleeve mounting plate 1711-3. The sleeve mounting plate 1711-3 is connected to the pricking control assembly 1713, so that the sleeve 1711-1 can slide up and down relative to the adapter 1711, so as to adjust the working state of the adapter 1711.

When the adapter 1711 is connected to the tip 01, the sleeve 1711-1 moves up under the action of an end face of the tip 01, so that the sleeve mounting plate 1711-3 can move up, thereby driving the pricking control assembly 1713 to move up. On the contrary, when the adapter 1711 exits the tip 01, the pricking control assembly 1713 is controlled to drive the sleeve mounting plate 1711-3 to descend, so that the sleeve 1711-1 descends along the adapter 1711, the tip 01 is gradually separated from the adapter 1711 until a lower end face of the sleeve 1711-1 is flush with a lower end face of the adapter 1711, and the tip 01 is completely separated from the adapter 1711.

The pricking control assembly 1713 is composed of a linear displacement mechanism and a driving mechanism. The linear displacement mechanism may specifically include a guide-rail and slider mechanism, a guide column and guide sleeve structure, and so on. The driving mechanism may specifically include an electrical driving mechanism such as a lead-screw motor, a step motor, a hydraulic cylinder, and an electric push rod.

In an embodiment, referring to Figure 19, the pricking control assembly 1713 includes a guide column 1713-1 having one end connected to the sleeve mounting plate 1711-3, a guide column connecting plate 1713-2 connected to the other end of the guide column 1713-1, and a lead-screw motor 1713-3. The guide column connecting plate 1713-2 is arranged below a motor shaft of the lead-screw motor 1713-3. The type, quantity, size, and position of the guide column 1713-1, and the type, model, position and the like of the lead-screw motor 1713-3, are determined by reference to the conventional technology according to the actual requirements, which are not described here.

Each of the reagent needle translation assembly 1714 and the reagent needle lifting assembly includes a linear displacement mechanism such as a guide-rail and slider mechanism, a synchronous belt mechanism, and a driving mechanism such as a step motor. Referring to Figure 18, the reagent needle translation assembly 1714 includes a synchronous belt mechanism and a driving motor. When the driving motor rotates, the driving motor drives a synchronous belt and the reagent needle connected to the synchronous belt to move along Y-axis direction.

When the reagent needle assembly 171 transfers the extraction reagent, the reagent needle translation assembly 1714 first drives the reagent needle to move along the Y-axis direction, so that the reagent needle directly faces the position of the tip 01 to be pricked. Then, the reagent needle lifting assembly drives the reagent needle to descend along the Y-axis, so that the lower end face of the adapter 1711 contacts the tip 01, the sleeve 1711-1 moves up under the action of the tip 01 and the adapter 1711 is exposed, the adapter 1711 is cooperatively connected to the tip 01 to complete the pricking of the tip 01. After the pricking is completed, the reagent needle lifting assembly drives the reagent needle to move up along Z-axis, the reagent needle translation assembly 1714 drives the reagent needle to move to the position directly above the reagent to be sucked, the reagent needle lifting assembly drives the reagent needle to move down along the Z-axis, and the pipetting control assembly controls the reagent pump 1712 to suck liquid through the tip 01 connected to the adapter 1711. After the suction is completed, the reagent needle lifting assembly drives the reagent needle to move up along the Z-axis, the reagent needle translation assembly 1714 drives the reagent needle to move to the position above the extraction reagent strip 03, the gantry assembly 173 drives the reagent needle to move to the position above the reaction chamber 031 along the X-axis direction, the reagent needle lifting assembly drives the reagent needle to descend along the Z-axis, the pipetting control assembly controls the reagent pump 1712 to inject the extraction reagent into the reaction chamber 031 through the adapter 1711. After the injection is completed, the reagent needle lifting assembly drives the reagent needle to move up along the Z-axis, the gantry assembly 173 drives the reagent needle to move to the discarding hole position, the pricking control assembly 1713 drives the sleeve 1711-1 to move down along a length direction of the adapter 1711 to separate the tip 01 from the adapter 1711to discard the tip 01.

Similarly, the reagent needle assembly 171 may be driven to inject the amplification reagent and a purified product into PCR tube 021 through a similar operation. In addition, the reagent needle assembly 171 can also tie the sealing plug 022 and cover the PCR tube 021 with the sealing plug 022.

In the embodiment, the reagent needle lifting assembly and the reagent needle translation assembly 1714 cooperate with the gantry assembly 173 to realize the adjustment of the spatial coordinates of the adapter 1711, so that the reagent needle assembly 171 is adapted to the pipetting of the extraction reagent, the pipetting of the amplification reagent, and the sealing of the PCR tube 021. The pricking control assembly 1713 realizes automatic pricking and discarding of the tip 01, thereby improving the pipetting efficiency.

In an embodiment, in order to facilitate the control system obtaining the working state of the reagent needle, the reagent needle assembly 171 further includes a tip taking detection assembly 1715 and a tip withdrawing detection assembly 1716. When the guide column connecting plate 1713-2 is moved up to the highest point, the tip taking detection assembly 1715 outputs a grasping success signal. When the lower end face of the sleeve 1711-1 is lowered to be flush with the lower end face of the adapter 1711, the tip withdrawing detection assembly 1716 outputs a withdrawing success signal. The structures, sizes, positions, and connection manners of the tip taking detection assembly 1715 and the tip withdrawing detection assembly 1716 are determined based on the actual detection requirements, which are not described here.

On the basis of the above embodiments, the sample adding needle assembly 172 includes multiple pipette pumps 1721 arranged in parallel, a pipette pump lifting assembly 1723 for driving the pipette pumps 1721 to go up and down, and a plunger lifting assembly 1722 for driving plungers of the pipette pumps 1721 to slide relative to pump bodies of the pipette pumps 1721. The pipette pumps 1721 are installed on the pipette pump lifting assembly 1723, and the plunger lifting assembly 1722 is connected to the plungers of the pipette pumps 1721.

Referring to Figure 20, the sample adding needle assembly 172 is connected to the gantry assembly 173 through a sample adding needle mounting seat, each of the pipette pumps 1721 is installed on a pipette pump mounting plate, and the plunger lifting assembly 1722 is installed on a liquid extraction and injection fixing plate. The plunger lifting assembly 1722 is arranged between the pipette pump mounting plate and the liquid extraction and injection fixing plate, and the pipette pump lifting assembly 1723 is arranged between the liquid extraction and injection fixing plate and the sample adding needle mounting seat.

The pipette pump 1721 includes the pump body and the plunger arranged in the pump body. A lower end of the pump body is configured to cooperate with the tip 01. The plunger is slidably connected to the pump body, so that the air pressure in the pump body is changed by changing the volume of the pump body to realize liquid extraction and liquid injection processes. The quantity, type, model and the like of the pipette pump 1721 are determined by reference to the conventional technology according to the actual requirements, which are not described here.

Each of the pipette pump lifting assembly 1723 and the plunger lifting assembly 1722 include a linear displacement mechanism and a driving mechanism. The linear displacement mechanism includes a guide-rail and slider mechanism, a synchronous belt mechanism and so on, and the driving mechanism includes a step motor, a lead-screw motor, a hydraulic cylinder, an electric push rod and so on.

In an embodiment, in order to control the plungers of multiple pipette pumps 1721 to move synchronously, the plunger lifting assembly 1722 is provided with a synchronization bar engaged with the plungers of the pipette pumps 1721.

When the sample adding needle assembly 172 performs pipetting, the gantry assembly 173 first drives the sample adding needle assembly 172 to move along the X-axis to the position above the disposable tip consumable box 12. The sample needle lifting assembly 1723 drives the pipette pump 1721 to descend along the Z-axis, so that the tip 01 is installed at the lower end of the pipette pump 1721. After the cooperation of the pipetting pump 1721 and the tip 01 is completed, the sample needle lifting assembly 1723 drives the pipette pump 1721 to rise along the Z-axis, the gantry assembly 173 drives the sample adding needle assembly 172 to move along the X-axis to the position above the reagent to be sucked, the sample adding needle assembly 1723 drives the pipette pump 1721 to descend along the Z-axis, and the plunger lifting assembly 172 drives the plunger to rise relative to the pump body along the Z-axis, so as to complete the liquid extraction. After the liquid extraction is completed, the sample adding needle lifting assembly 1723 drives the pipette pump 1721 to rise along the Z-axis, the gantry assembly 173 drives the sample adding needle assembly 172 to move along the X-axis to the position where the liquid is to be added, the sample adding needle lifting assembly 1723 drives the pipette pump 1721 to descend along the Z-axis, and the plunger lifting assembly 172 drives the plunger to descend relative to the pump body along the Z-axis, so as to complete the liquid injection. After the liquid injection is completed, the sample adding needle lifting assembly 1723 drives the pipette pump 1721 to rise along the Z-axis, the gantry assembly 173 drives the sample adding needle assembly 172 to move along the X-axis to the discarding hole position, the sample adding needle lifting assembly 1723 drives the pipette pump 1721 to descend along the Z-axis, and the plunger lifting assembly 172 drives the plunger to descend relative to the pump body along the Z-axis, so that an ejector pin at a bottom of the plunger is exposed and presses the tip 01 to separate the tip 01 from the pipette pump 1721, thereby completing the automatic discarding of the tip 01.

In the embodiment, multiple pipette pumps 1721 are arranged in parallel in the sample adding needle assembly 172, and the synchronization bar can drive the plungers of the multiple pipette pumps 1721 to move synchronously, which realizes synchronous tip installation, synchronous pipetting, and synchronous tip discarding in multiple channels, avoids a large number of repetitive operations when multiple identical reagents are provided to the same samples for detection, saves the operation time, and greatly accelerates the detection speed. The sample adding needle assembly 172 has a compact structure, which saves the installation space, and facilitates miniaturization of the DNA and/or RNA nucleic acid co-extraction and detection system.

In an embodiment, the sample adding needle assembly 172 further includes a liquid extraction and injection detection assembly 1724 for detecting the working state of the pipette pump 1721, and a Z-axis origin detection assembly 1725 for detecting whether the pipette pump lifting assembly 1723 is reset. The types, installation manners, and positions of the liquid extraction and injection detection assembly 1724 and the Z-axis origin detection assembly 1725 are determined based on the actual detection requirements, which are not described here.

On the basis of the above embodiments, the PCR transfer device 21 includes a gripper assembly 211 for grasping or releasing the PCR tube 021, an cover opening assembly 212 for opening or closing an amplification cover 314 of the PCR amplification device 31, an X-axis assembly 213 for driving the gripper assembly 211 and the cover opening assembly 212 to move along the X-axis direction, a Y-axis assembly 214 for driving the gripper assembly 211 and the cover opening assembly 212 to move along the Y-axis direction, and a Z-axis assembly 215 for driving the gripper assembly 211 and the cover opening assembly 212 to move along the Z-axis direction. The gripper assembly 211 and the cover opening assembly 212 are slidably connected to the Z-axis assembly 215, the Z-axis assembly 215 is slidably connected to the Y-axis assembly 214, and the Y-axis assembly 214 is slidably connected to the X-axis assembly 213.

It should be noted that the gripper assembly 211 and the cover opening assembly 212 are slidably connected to the Z-axis assembly 215, that is, there is no direct connection between the gripper assembly 211 and the cover opening assembly 212.

Each of the X-axis assembly 213, the Y-axis assembly 214, and the Z-axis assembly 215 includes a linear displacement mechanism and a driving mechanism. The linear displacement mechanism is one of or a combination of multiple linear displacement mechanisms such as a synchronous belt mechanism, and a guide-rail and slider mechanism. The driving mechanism includes a conventional driving mechanism such as a step motor, an electric push rod, and a hydraulic cylinder. The specific structures, sizes, positions, and connection manners of the X-axis assembly 213, the Y-axis assembly 214, and the Z-axis assembly 215 are determined based on the actual detection requirements, which are not described here.

When the PCR transfer device 21 works, the X-axis assembly 213 and the Y-axis assembly 214 drive the Z-axis assembly 215 to move to the positon directly above an amplification heating block 311 of the PCR amplification device 31. The Z-axis assembly 215 drives the cover opening assembly 212 to move downwards along the Z-axis, so that the cover opening assembly 212 engages with and is fixed to the amplification cover 314. The Y-axis assembly 214 drives the gripper assembly 211 and the cover opening assembly 212 to move along the Y-axis direction, so that the amplification cover 314 is separated from the amplification heating block 311 of the PCR amplification device 31 to complete the cover opening operation.

The Z-axis assembly 215 drives the gripper assembly 211 to descend to a low position (a grasping position) and the cover opening assembly 212 to rise to a high position. The X-axis assembly 213 and the Y-axis assembly 214 drive the gripper assembly 211 to move to the position of the PCR consumable box 13, and the gripper assembly 211 grasps the PCR tube 021. The X-axis assembly 213 and the Y-axis assembly 214 drive the gripper assembly 211 to move to the amplification heating block 311 of the PCR amplification device 31, and the gripper assembly 211 releases the PCR tube 021, so that the PCR tube 021 is placed into an amplification hole of the amplification heating block 311.

After the reaction chamber 031 is filled with the PCR tubes 021, the Z-axis assembly 215 drives the cover opening assembly 212 to descend to the lower position and the gripper assembly 211 to rise to the high position, and the cover opening assembly 212 closes the amplification cover 314.

After the amplification reaction is completed, the PCR transfer device 21 takes out the reacted PCR tube 021 according to the similar operation as described above, discards the PCR tube 021 to the discarding position, and finally closes the amplification cover 314.

In the embodiment, the PCR transfer device 21 realizes a three-dimensional spatial movement of the PCR tube 021 and the automatic operation of the opening and closing of the amplification cover 314. The gripper assembly 211 and the opening cover assembly 212 are controlled by the same motor, and move up and down alternately, which is beneficial for reducing the volume of the device, improving the grasping efficiency, and effectively improving the operating efficiency and the use effect of the PCR transfer device 21.

On the basis of the above embodiments, the gripper assembly 211 includes a gripper base plate, a gripper movable plate parallel to the bottom of the gripper base plate, a gripper for grasping the PCR tube 021, and a retraction drive assembly for controlling the opening or retraction of the gripper. The retraction drive assembly is arranged on the gripper base plate, and a telescopic end of the retraction drive assembly passes through the gripper base plate to be connected to the gripper movable plate, so as to drive the gripper movable plate to rise or descend. The gripper base plate and the gripper movable plate are connected to the retraction drive assembly. The gripper opens when the gripper movable plate descends, and the gripper retracts when the gripper movable plate rises.

The shapes, structures, sizes, materials, and positions of the gripper base plate, the gripper movable plate, the gripper, and the retraction drive assembly are determined based on actual situations and actual requirements.

When the retraction drive assembly moves telescopically, the telescopic end of the retraction drive assembly drives the gripper movable plate to descend and drives the gripper to open, thereby facilitating the gripper to grasp the PCR tube 021. When the telescopic end of the retraction drive assembly drives the gripper movable plate to rise, the gripper movable plate drives the gripper to retract and close, so that the gripper can grasp the PCR tube 021. The X-axis assembly 213, the Y-axis assembly 214, and the Z-axis assembly 215 drive the PCR tube 021 to move to the desired position after the gripper grasps the PCR tube 021.

In the embodiment, the PCR tube transfer device 21 effectively improves the grasping and transferring efficiency of the PCR tube 021, and ensures the accuracy of the grasping and transferring operation of the PCR tube 021. The structure is simple, the operation is convenient, and the popularization is convenient.

In order to prevent the nucleic acid sample in the PCR tube 021 from being contaminated during the transferring process, the PCR tube 021 is sealed with the sealing plug 022, and the sealing plug 022 is removed after the PCR tube 021 is transferred into place.

In an embodiment, the gripper assembly 211 further includes a guide column for engaging with the sealing plug 022 at the top of the PCR tube 021 and an ejector sleeve, and the ejector sleeve is configured to eject the sealing plug 022. The guide column penetrates through the gripper movable plate to be fixedly connected to the gripper base plate, and the guide column is located at the axis of the gripper. The ejector sleeve is arranged on an outer periphery of the guide column, and the ejector sleeve is fixedly connected to the gripper movable plate.

When the retraction drive assembly drives the gripper movable plate to descend, the gripper is opened and the ejector sleeve fixed on the gripper movable plate also descends. The ejector sleeve ejects back the sealing plug 022 at the top of the PCR tube 021, so as to realize the ejection of the PCR tube 021. When the retraction drive assembly drives the gripper movable plate to rise, the gripper retracts and is closed, and the guide column is fixed. The ejector sleeve moves up with the gripper movable plate. At this time, the guide column is exposed and inserted into the sealing plug 022, so that the guide column and the sealing plug 022 are engaged with each other and fixed, thereby realizing the grasping of the PCR tube 021. After the PCR tube 021 is grasped by the gripper, the X-axis assembly 213, the Y-axis assembly 214, and the Z-axis assembly 215 drive the PCR tube 021 to move to the desired position.

The retraction drive assembly drives the movements of the gripper and the ejector sleeve, so that the gripper and the guide column can cooperate to grasp the PCR tube 021, and the ejector sleeve can effectively eject the PCR tube 021, which effectively improves the operational efficiency of the device and prevents contamination caused by manual intervention. In addition, the volume device is reduced, and the space is saved.

In an embodiment, the gripper assembly 211 further includes an ejecting-back detection assembly for detecting whether the sealing plug 022 has been successfully ejected back. The structure, size, position, etc. of the push detection assembly are determined based on the actual requirements, which are not described here.

On the basis of the above embodiments, the PCR amplification device 31 includes an amplification heating block 311, an amplification heating plate 312, an amplification radiator 313, an amplification cover 314 for covering the amplification heating block 311, and an amplification cooling assembly 315 for improving the heat dissipation efficiency of the amplification radiator 313. The amplification heating block 311 has at least two amplification holes for placing the PCR tubes 021. The amplification cover 314, the amplification heating block 311, the amplification heating plate 312 and the amplification radiator 313 are sequentially arranged from top to bottom.

Referring to Figure 25, the number of amplification heating blocks 311 may be one, two, or more, and each amplification heating block 311 may have multiple amplification holes. Each amplification heating block 311 corresponds to one amplification heating plate 312 for heating to reduce device costs. The amplification cooling assembly 315 is arranged below the amplification radiator 313 to improve the heat dissipation efficiency of the amplification radiator 313, thereby accelerating the rise and decrease speed of the temperature of the PCR amplification and reducing amplification time.

In an embodiment, the amplification heating block 311 is provided with an amplification temperature sensor for detecting the temperature of the amplification heating block 311 in real time, so that the detector can master the temperature of the amplification heating block 311 in real time, thereby effectively controlling the heating temperature.

The shapes, structures, sizes, materials, positions, etc. of the amplification heating block 311, the amplification heating plate 312, the amplification radiator 313, the amplification cover 314, the amplification cooling assembly 315, and the amplification temperature sensor are determined according to the actual requirements with reference to the conventional technology.

When the PCR amplification device 31 is working, the amplification cover 314 is opened firstly, and the PCR tube 021 containing the nucleic acid sample is placed into the amplification hole of the amplification heating block 311. After the placement of the PCR tube 021 is completed, the amplification cover 314 is covered, and the amplification heating plate 312 is used to heat the amplification heating block 311, so as to facilitate PCR amplification of the nucleic acid sample.

When multiple detection items are performed on a same nucleic acid sample, a single nucleic acid is required to be divided into multiple parts for amplification. One amplification heating block 311 of the PCR amplification device 31 according to the embodiment has multiple amplification holes, and the heating temperature of each amplification heating block 311 remains consistent, which is beneficial to ensuring the same amplification condition of the same nucleic acid sample and reduces the deviation of the amplification result of the sample caused by different amplification temperatures. After the amplification cover 314 covers the amplification heating block 311, the amplification heating block 311 and the sample can be thermally insulated during the amplification process, so that the temperature rise speed is fast and the amplification efficiency is high. In addition, the amplification cooling assembly 315 effectively improves the heat dissipation efficiency of the amplification radiator 313, thereby accelerating the rise and decrease speed of the temperature of the sample amplification, reducing the amplification time of the sample, and improving the amplification efficiency of the sample.

In an embodiment, the amplification cover 314 includes a frame and a top cover that can slide horizontally along the frame. The top cover and the amplification heating block 311 are arranged in one-to-one correspondence. The frame is provided with a cover-opening sensor for detecting whether the top cover is opened, and the top cover is provided with a cover-opening baffle for triggering the cover-opening sensor. The structures, materials, sizes, positions, and the like of the frame, the top cover, the cover-opening sensor, and the cover-opening baffle are determined based on the actual requirements, which are not be described here.

When the cover is covered, the position of the cover-opening baffle corresponds to the position of the cover-opening sensor, and the cover-opening baffle triggers the cover-opening sensor. When the cover is opened, the cover-opening baffle slides with the top cover, the cover-opening baffle is separated from the cover-opening sensor, and the cover-opening baffle does not trigger the cover-opening sensor.

Real time fluorescence quantitative detection PCR technology refers to a method where a fluorescent group is added to a PCR reaction system, the entire PCR process is monitored in real time by using fluorescence signal accumulation, and finally a quantitative analysis is performed on an unknown template through a standard curve. This technology uses fluorescence signals to detect the PCR product, so that the sensitivity of polymerase chain reaction is improved, and one data is collected per cycle of the polymerase chain reaction. Therefore, a real-time nucleic acid sample amplification curve can be established and the CT value can be accurately determined, so that the initial nucleic acid copy number is determined based on the CT value, which realized nucleic acid quantitative detection.

On the basis of the above embodiments, the fluorescence detection device 32 includes a light source assembly 321 for providing a fluorescent light source, a fluorescence detection assembly 322 for quantitative detection, a light source optical fiber 323, and a fluorescence detection optical fiber 324. One side of the amplification hole is connected to one end of the light source optical fiber 323, and the other side of the amplification hole is connected to one end of the fluorescence detection optical fiber 324. The other end of the light source optical fiber 323 is connected to the light source assembly 321, and the other end of the fluorescence detection optical fiber 324 is connected to the fluorescence detection assembly 322.

The structures, materials, sizes, positions, connection manners and the like of the light source assembly 321, the fluorescence detection assembly 322, the light source optical fiber 323, and the fluorescence detection optical fiber 324 are determined based on the actual detection requirements.

In an embodiment, the light source optical fiber 323 and the fluorescence detection optical fiber 324 are respectively connected to both sides of the amplification heating block 311. An angle between the light source optical fiber 323 and the fluorescence detection optical fiber 324 is 90 degrees to ensure accurate and reliable positioning of each of the fibers, which is beneficial to the accuracy of the light measurement value.

It should be noted that, "first" and "second" in the first magnet, the second magnet, the first magnetic attraction surface 1621-1 and the second magnetic attraction surface 1621-2 mentioned in the present application, are only used to distinguish different positions, without the limitation of the order.

The various embodiments in the specification are described in a progressive manner, and each embodiment focuses on the differences from other embodiments. The same and similar parts between the various embodiments can be referred to each other.

The DNA and/or RNA nucleic acid co-extraction and detection system according to the present application have been introduced in detail above. Specific embodiments are used herein to illustrate the principles and the implementations of the present application, and the descriptions of the above embodiments are only used to help understand methods and core ideas of the present application. It should be pointed out that for those skilled in the art, some improvements and modifications may be made to the present application without departing from the principles of the present application, and these improvements and modifications shall fall within the protection scope of the claims of the present application.

## Claims

1. A DNA and/or RNA nucleic acid co-extraction and detection system, comprising a nucleic acid extraction system, a sealing transfer mixing system, a nucleic acid detection system, and a control system, wherein the nucleic acid extraction system, the sealing transfer mixing system, and the nucleic acid detection system are connected to the control system;
the nucleic acid extraction system comprises a basket (11) for loading an extraction reagent strip (03) and a sample tube (04), a consumable supply module, a kit loading module (14), a barcode scanning device (15) for automatically inputting barcode information, a magnetic-attraction incubation device (16) and an automatic sample adding device (17) for transferring liquid into a reaction chamber (031) of the extraction reagent strip (03) or a PCR tube (021) and sealing the PCR tube (021);
the sealing transfer system comprises a PCR transfer device (21) and a high-speed mixing device (22), and the PCR transfer device (21) is configured for grasping the PCR tube (021) and transferring the PCR tube (021) to the high-speed mixing device (22) for high-speed mixing; and
the nucleic acid detection system comprises a PCR amplification device (31) and a fluorescence detection device (32).

2. The DNA and/or RNA nucleic acid co-extraction and detection system according to claim 1, wherein the basket (11) comprises a basket cover plate (112), a basket bottom plate (111) for holding the extraction reagent strip (03), and a sample holder (113) for holding the sample tube (04), the sample holder (113) is detachably connected to the basket bottom plate (111); and
the basket cover plate (112) is rotatably connected to the basket bottom plate (111) to limit a displacement of the extraction reagent strip (03) in a height direction when the basket cover plate (112) contacts the basket bottom plate (111).

3. The DNA and/or RNA nucleic acid co-extraction and detection system according to claim 1, wherein the barcode scanning device (15) comprises a mounting support (151), a scanning assembly (153), a pusher assembly (154) for pushing the extraction reagent strip (03) between a scanning position and a standby position, a pusher moving assembly (152) for driving the pusher assembly (154) to move along a length direction of the mounting support (151) and a detection assembly (155), the detection assembly (155) is configured to determine whether there is an extraction reagent strip (03) and a sample tubes (04) in a current channel, the detection assembly (155) is in signal connection with the pusher moving assembly (152), and the scanning assembly (153) and the detection assembly (155) are mounted on a mounting base plate of the pusher assembly (154).

4. The DNA and/or RNA nucleic acid co-extraction and detection system according to claim 1, wherein the magnetic-attraction incubation device (16) comprises an incubation module (161) for heating the reaction chamber (031), a magnetic attraction module (162) for magnetically attracting the reaction chamber (031), and a driving assembly (163), and, both the incubation module (161) and the magnetic attraction module (162) are connected to the driving assembly (163), the driving assembly (163) is configured to drive the incubation module (161) and the magnetic attraction module (162) alternately approach the reaction chamber (031).

5. The DNA and/or RNA nucleic acid co-extraction and detection system according to claim 4, wherein the incubation module (161) comprises a heating block (1611) having a reagent groove (1611-1), a heating assembly (1612) contacting an inner wall of the reagent groove (1611-1), a heat-dissipation assembly (1613) provided at a bottom of the heating block (1611), and a temperature sensor (1614) for detecting the temperature of the heating block (1611), and, the temperature sensor (1614) is connected to the heating block (1611).

6. The DNA and/or RNA nucleic acid co-extraction and detection system according to claim 4, wherein the magnetic attraction module (162) comprises a magnet (1621) and a rotating assembly (1622) for driving the magnet (1621) to rotate, the rotating assembly (1622) is connected to the magnet (1621);
the magnet (1621) comprises a first magnetic attraction surface (1621-1) and a second magnetic attraction surface (1621-2), and the first magnetic attraction surface (1621-1) and the second magnetic attraction surface (1621-2) have different areas and are configured to attract magnetic beads in the reaction chamber (031).

7. The DNA and/or RNA nucleic acid co-extraction and detection system according to claim 1, wherein the PCR transfer device (21) comprises a gripper assembly (211) for grasping or releasing the PCR tube (021), an cover opening assembly (212) for opening or closing the amplification cover (314) of the PCR amplification device (31), an X-axis assembly (213) for driving the gripper assembly (211) and the cover opening assembly (212) to move along an X-axis direction, a Y-axis assembly (214) for driving the gripper assembly (211) and the cover opening assembly (212) to move along a Y-axis direction, and a Z-axis assembly (215) for driving the gripper assembly (211) and the cover opening assembly (212) to move along a Z-axis direction; and
the gripper assembly (211) and the cover opening assembly (212) are slidably connected to the Z-axis assembly (215), the Z-axis assembly (215) is slidably connected to the Y-axis assembly (214), and the Y-axis assembly (214) is slidably connected to the X-axis assembly (213).

8. The DNA and/or RNA nucleic acid co-extraction and detection system according to claim 7, wherein the gripper assembly (211) comprises a gripper base plate, a gripper movable plate parallel to a bottom of the gripper base plate, a gripper for grasping the PCR tube (021), and a retraction drive assembly for controlling opening or retraction of the gripper, the retraction drive assembly is arranged on the gripper base plate, and a telescopic end of the retraction drive assembly penetrates through the gripper base plate to be connected to the gripper movable plate, so as to drive the gripper movable plate to rise or descend; and
the gripper bottom plate and the gripper movable plate are connected to the retraction drive assembly, the gripper opens when the gripper movable plate descends, and the gripper retracts when the gripper movable plate rises.

9. The DNA and/or RNA nucleic acid co-extraction and detection system according to claim 1, wherein the PCR amplification device (31) comprises an amplification heating block (311), an amplification heating plate (312), an amplification radiator (313), an amplification cover (314) for covering the amplification heating block (311), and an amplification cooling assembly (315) for improving heat dissipation efficiency of the amplification radiator (313), the amplification heating block (311) has at least two amplification holes for placing the PCR tubes (021), and, the amplification cover (314), the amplification heating block (311), the amplification heating plate (312), and the amplification radiator (313) are sequentially arranged from top to bottom.

10. The DNA and/or RNA nucleic acid co-extraction and detection system according to claim 9, wherein the fluorescence detection device (32) comprises a light source assembly (321) for providing a fluorescent light source, a fluorescence detection assembly (322) for quantitative detection, a light source optical fiber (323), and a fluorescence detection optical fiber (324), and, one side of the amplification hole is connected to one end of the light source optical fiber (323), and the other side of the amplification hole is connected to one end of the fluorescence detection optical fiber (324); and
the other end of the light source optical fiber (323) is connected to the light source assembly (321), and the other end of the fluorescence detection optical fiber (324) is connected to the fluorescence detection assembly (322).

11. The DNA and/or RNA nucleic acid co-extraction and detection system according to any one of claims 1 to 10, wherein the automatic sample adding device (17) comprises a reagent needle assembly (171) for extraction and injection of a reagent, a sample adding needle assembly (172) for extraction and injection of sample infusion and a gantry assembly (173), the reagent needle assembly (171), the sample adding needle assembly (172) and the gantry assembly (173) are connected to the control system; and
the reagent needle assembly (171) and the sample adding needle assembly (172) are respectively arranged on both sides of the gantry assembly (173), and the gantry assembly (173) is configured to move along the basket (11), the consumable supply module, and the kit loading module (14).

12. The DNA and/or RNA nucleic acid co-extraction and detection system according to claim 11, wherein the reagent needle assembly (171) comprises a reagent needle, a pricking control assembly (1713), a pipetting control assembly, a reagent needle lifting assembly, and a reagent needle translation assembly (1714), and, the reagent needle comprises an adapter (1711) and a reagent pump (1712) communicating with the adapter (1711), the reagent pump (1712) is connected to the pipetting control assembly, and the adapter (1711) is connected to the pricking control assembly (1713) for the pricking control assembly (1713) to control the adapter (1711) to prick or exit a tip (01) or a sealing plug (022); and
the reagent needle translation assembly (1714) is configured to drive the reagent needle to move along a direction perpendicular to a movement direction of the gantry assembly (173) in a horizontal plane, and the reagent needle lifting assembly is configured to drive the reagent needle to move along a vertical direction.

13. The DNA and/or RNA nucleic acid co-extraction and detection system according to claim 11, wherein the sample adding needle assembly (172) comprises a plurality of pipette pumps (1721) provided in parallel, a pipette pump lifting assembly (1723) for driving the plurality of pipette pumps (1721) to go up and down, and a plunger lifting assembly (1722) for driving plungers of the plurality of pipette pumps (1721) to slide relative to pump bodies of the plurality of pipette pumps (1721), the plurality of pipette pumps (1721) are installed on the pipette pump lifting assembly (1723), and the plunger lifting assembly (1722) is connected to the plungers of the plurality of pipette pumps (1721).

## Patentansprüche

1. DNA- und/oder RNA-Nukleinsäure-Koextraktions- und -Nachweissystem, umfassend ein Nukleinsäureextraktionssystem, ein Verschließ-, Transfer- und Mischsystem, ein Nukleinsäurenachweissystem und ein Steuersystem, wobei das Nukleinsäureextraktionssystem, das Verschließ-, Transfer- und Mischsystem und das Nukleinsäurenachweissystem mit dem Steuersystem verbunden sind;
das Nukleinsäureextraktionssystem einen Korb (11) zum Laden eines Extraktionsreagenzstreifens (03) und eines Probenröhrchens (04), ein Verbrauchsmaterialversorgungsmodul, ein Kit-Lademodul (14), eine Barcode-Scanvorrichtung (15) zum automatischen Eingeben von Barcodeinformationen, eine Magnetanziehungs-Inkubationsvorrichtung (16) und eine automatische Probenzugabevorrichtung (17) zum Transferieren von Flüssigkeit in eine Reaktionskammer (031) des Extraktionsreagenzstreifens (03) oder ein PCR-Röhrchen (021) und zum Verschließen des PCR-Röhrchens (021) umfasst;
das Verschließ-, Transfersystem eine PCR-Transfervorrichtung (21) und eine Hochgeschwindigkeitsmischvorrichtung (22) umfasst und die PCR-Transfervorrichtung (21) dazu ausgebildet ist, das PCR-Röhrchen (021) zu greifen und das PCR-Röhrchen (021) zur Hochgeschwindigkeitsmischvorrichtung (22) zur Hochgeschwindigkeitsmischung zu transferieren; und
das Nukleinsäurenachweissystem eine PCR-Amplifikationsvorrichtung (31) und eine Fluoreszenzdetektionsvorrichtung (32) umfasst.

2. DNA- und/oder RNA-Nukleinsäure-Koextraktions- und -Nachweissystem nach Anspruch 1, wobei der Korb (11) eine Korbabdeckplatte (112), eine Korbbodenplatte (111) zum Halten des Extraktionsreagenzstreifens (03) und einen Probenhalter (113) zum Halten des Probenröhrchens (04) umfasst, der Probenhalter (113) lösbar mit der Korbbodenplatte (111) verbunden ist; und
die Korbabdeckplatte (112) drehbar mit der Korbbodenplatte (111) verbunden ist, um eine Verschiebung des Extraktionsreagenzstreifens (03) in einer Höhenrichtung zu begrenzen, wenn die Korbabdeckplatte (112) die Korbbodenplatte (111) kontaktiert.

3. DNA- und/oder RNA-Nukleinsäure-Koextraktions- und -Nachweissystem nach Anspruch 1, wobei die Barcode-Scanvorrichtung (15) eine Montagehalterung (151), eine Scananordnung (153), eine Schieberanordnung (154) zum Schieben des Extraktionsreagenzstreifens (03) zwischen einer Scanposition und einer Bereitschaftsposition, eine Schieberbewegungsanordnung (152) zum Antreiben der Schieberanordnung (154), damit diese sich entlang einer Längsrichtung der Montagehalterung (151) bewegt, und eine Detektionsanordnung (155) umfasst, die Detektionsanordnung (155) dazu ausgebildet ist, zu bestimmen, ob sich ein Extraktionsreagenzstreifen (03) und ein Probenröhrchen (04) in einem aktuellen Kanal befinden, die Detektionsanordnung (155) signalmäßig mit der Schieberbewegungsanordnung (152) verbunden ist und die Scananordnung (153) und die Detektionsanordnung (155) auf einer Montagegrundplatte der Schieberanordnung (154) montiert sind.

4. DNA- und/oder RNA-Nukleinsäure-Koextraktions- und -Nachweissystem nach Anspruch 1, wobei die Magnetanziehungs-Inkubationsvorrichtung (16) ein Inkubationsmodul (161) zum Erhitzen der Reaktionskammer (031), ein Magnetanziehungsmodul (162) zum magnetischen Anziehen der Reaktionskammer (031) und eine Antriebsanordnung (163) umfasst und sowohl das Inkubationsmodul (161) als auch das Magnetanziehungsmodul (162) mit der Antriebsanordnung (163) verbunden sind, die Antriebsanordnung (163) dazu ausgebildet ist, das Inkubationsmodul (161) und das Magnetanziehungsmodul (162) abwechselnd an die Reaktionskammer (031) heranzuführen.

5. DNA- und/oder RNA-Nukleinsäure-Koextraktions- und -Nachweissystem nach Anspruch 4, wobei das Inkubationsmodul (161) einen Heizblock (1611) mit einer Reagenzrille (1611-1), eine Heizanordnung (1612), die eine Innenwand der Reagenzrille (1611-1) kontaktiert, eine Wärmeableitungsanordnung (1613), die an einem Boden des Heizblocks (1611) bereitgestellt ist, und einen Temperatursensor (1614) zum Detektieren der Temperatur des Heizblocks (1611) umfasst und der Temperatursensor (1614) mit dem Heizblock (1611) verbunden ist.

6. DNA- und/oder RNA-Nukleinsäure-Koextraktions- und -Nachweissystem nach Anspruch 4, wobei das Magnetanziehungsmodul (162) einen Magneten (1621) und eine Drehanordnung (1622) zum Versetzen des Magneten (1621) in Drehung umfasst, die Drehanordnung (1622) mit dem Magneten (1621) verbunden ist;
der Magnet (1621) eine erste Magnetanziehungsfläche (1621-1) und eine zweite Magnetanziehungsfläche (1621-2) umfasst und die erste Magnetanziehungsfläche (1621-1) und die zweite Magnetanziehungsfläche (1621-2) unterschiedliche Flächen aufweisen und dazu ausgebildet sind, magnetische Perlen in der Reaktionskammer (031) anzuziehen.

7. DNA- und/oder RNA-Nukleinsäure-Koextraktions- und -Nachweissystem nach Anspruch 1, wobei die PCR-Transfervorrichtung (21) eine Greiferanordnung (211) zum Greifen oder Freigeben des PCR-Röhrchens (021), eine Deckelöffnungsanordnung (212) zum Öffnen oder Schließen der Amplifikationsabdeckung (314) der PCR-Amplifikationsvorrichtung (31), eine X-Achsen-Anordnung (213) zum Antreiben der Greiferanordnung (211) und der Deckelöffnungsanordnung (212), damit diese sich entlang einer X-Achsen-Richtung bewegen, eine Y-Achsen-Anordnung (214) zum Antreiben der Greiferanordnung (211) und der Deckelöffnungsanordnung (212), damit diese sich entlang einer Y-Achsen-Richtung bewegen, und eine Z-Achsen-Anordnung (215) zum Antreiben der Greiferanordnung (211) und der Deckelöffnungsanordnung (212), damit diese sich entlang einer Z-Achsen-Richtung bewegen, umfasst; und
die Greiferanordnung (211) und die Deckelöffnungsanordnung (212) gleitend mit der Z-Achsen-Anordnung (215) verbunden sind, die Z-Achsen-Anordnung (215) gleitend mit der Y-Achsen-Anordnung (214) verbunden ist und die Y-Achsen-Anordnung (214) gleitend mit der X-Achsen-Anordnung (213) verbunden ist.

8. DNA- und/oder RNA-Nukleinsäure-Koextraktions- und -Nachweissystem nach Anspruch 7, wobei die Greiferanordnung (211) eine Greifergrundplatte, eine Greiferbewegungsplatte parallel zu einem Boden der Greifergrundplatte, einen Greifer zum Greifen des PCR-Röhrchens (021) und eine Rückzugsantriebsanordnung zum Steuern des Öffnens oder Zurückziehens des Greifers umfasst, die Rückzugsantriebsanordnung auf der Greifergrundplatte angeordnet ist und ein teleskopisches Ende der Rückzugsantriebsanordnung durch die Greifergrundplatte hindurchgeht, um mit der Greiferbewegungsplatte verbunden zu sein, um die Greiferbewegungsplatte zum Ansteigen oder Absenken anzutreiben; und
die Greiferbodenplatte und die Greiferbewegungsplatte mit der Rückzugsantriebsanordnung verbunden sind, sich der Greifer öffnet, wenn die Greiferbewegungsplatte absinkt, und sich der Greifer zurückzieht, wenn die Greiferbewegungsplatte ansteigt.

9. DNA- und/oder RNA-Nukleinsäure-Koextraktions- und -Nachweissystem nach Anspruch 1, wobei die PCR-Amplifikationsvorrichtung (31) einen Amplifikationsheizblock (311), eine Amplifikationsheizplatte (312), einen Amplifikationskühler (313), eine Amplifikationsabdeckung (314) zum Abdecken des Amplifikationsheizblocks (311) und eine Amplifikationskühlanordnung (315) zur Verbesserung der Wärmeableitungseffizienz des Amplifikationskühlers (313) umfasst, der Amplifikationsheizblock (311) mindestens zwei Amplifikationslöcher zum Platzieren der PCR-Röhrchen (021) aufweist und die Amplifikationsabdeckung (314), der Amplifikationsheizblock (311), die Amplifikationsheizplatte (312) und der Amplifikationskühler (313) nacheinander von oben nach unten angeordnet sind.

10. DNA- und/oder RNA-Nukleinsäure-Koextraktions- und -Nachweissystem nach Anspruch 9, wobei die Fluoreszenzdetektionsvorrichtung (32) eine Lichtquellenanordnung (321) zum Bereitstellen einer fluoreszierenden Lichtquelle, eine Fluoreszenzdetektionsanordnung (322) zur quantitativen Detektion, eine Lichtquellen-Lichtleitfaser (323) und eine Fluoreszenzdetektions-Lichtleitfaser (324) umfasst und eine Seite des Amplifikationslochs mit einem Ende der Lichtquellen-Lichtleitfaser (323) verbunden ist und die andere Seite des Amplifikationslochs mit einem Ende der Fluoreszenzdetektions-Lichtleitfaser (324) verbunden ist; und
das andere Ende der Lichtquellen-Lichtleitfaser (323) mit der Lichtquellenanordnung (321) verbunden ist und das andere Ende der Fluoreszenzdetektions-Lichtleitfaser (324) mit der Fluoreszenzdetektionsanordnung (322) verbunden ist.

11. DNA- und/oder RNA-Nukleinsäure-Koextraktions- und -Nachweissystem nach einem der Ansprüche 1 bis 10, wobei die automatische Probenzugabevorrichtung (17) eine Reagenznadelanordnung (171) zur Extraktion und Injektion eines Reagenzes, eine Probenzugabenadelanordnung (172) zur Extraktion und Injektion von Probeninfusion und eine Portalbaugruppe (173) umfasst, die Reagenznadelanordnung (171), die Probenzugabenadelanordnung (172) und die Portalbaugruppe (173) mit dem Steuersystem verbunden sind; und
die Reagenznadelanordnung (171) und die Probenzugabenadelanordnung (172) jeweils an beiden Seiten der Portalbaugruppe (173) angeordnet sind, und die Portalbaugruppe (173) dazu ausgebildet ist, sich entlang des Korbs (11), des Verbrauchsmaterialversorgungsmoduls und des Kit-Lademoduls (14) zu bewegen.

12. DNA- und/oder RNA-Nukleinsäure-Koextraktions- und -Nachweissystem nach Anspruch 11, wobei die Reagenznadelanordnung (171) eine Reagenznadel, eine Einstechsteuerungsanordnung (1713), eine Pipettiersteuerungsanordnung, eine Reagenznadel-Hebeanordnung und eine Reagenznadel-Translationsanordnung (1714) umfasst und die Reagenznadel einen Adapter (1711) und eine mit dem Adapter (1711) in Verbindung stehende Reagenzpumpe (1712) umfasst, die Reagenzpumpe (1712) mit der Pipettiersteuerungsanordnung verbunden ist und der Adapter (1711) mit der Einstechsteuerungsanordnung (1713) verbunden ist, damit die Einstechsteuerungsanordnung (1713) den Adapter (1711) steuert, in eine Spitze (01) oder einen Verschlussstopfen (022) einzustechen oder diese(n) zu verlassen; und
die Reagenznadel-Translationsanordnung (1714) dazu ausgebildet ist, die Reagenznadel entlang einer Richtung zu bewegen, die in einer horizontalen Ebene senkrecht zu einer Bewegungsrichtung der Portalbaugruppe (173) verläuft, und die Reagenznadel-Hebeanordnung dazu ausgebildet ist, die Reagenznadel entlang einer vertikalen Richtung zu bewegen.

13. DNA- und/oder RNA-Nukleinsäure-Koextraktions- und -Nachweissystem nach Anspruch 11, wobei die Probenzugabenadelanordnung (172) eine Vielzahl von parallel bereitgestellten Pipettenpumpen (1721), eine Pipettenpumpen-Hebeanordnung (1723) zum Antreiben der Vielzahl von Pipettenpumpen (1721) zum Auf- und Abbewegen und eine Kolben-Hebeanordnung (1722) zum Antreiben von Kolben der Vielzahl von Pipettenpumpen (1721) zum Gleiten relativ zu Pumpenkörpern der Vielzahl von Pipettenpumpen (1721) umfasst, die Vielzahl von Pipettenpumpen (1721) auf der Pipettenpumpen-Hebeanordnung (1723) installiert ist und die Kolben-Hebeanordnung (1722) mit den Kolben der Vielzahl von Pipettenpumpen (1721) verbunden ist.

## Revendications

1. Système de détection et de co-extraction d'acides nucléiques d'ADN et/ou d'ARN, comprenant un système d'extraction d'acides nucléiques, un système de scellement, de transfert et de mélange, un système de détection d'acides nucléiques, et un système de commande, dans lequel le système d'extraction d'acides nucléiques, le système de scellement, de transfert et de mélange, et le système de détection d'acides nucléiques sont reliés au système de commande ;
le système d'extraction d'acides nucléiques comprend un panier (11) permettant de charger une bandelette (03) réactive d'extraction et un tube (04) d'échantillon, un module d'alimentation en consommables, un module (14) de chargement de kit, un dispositif (15) de balayage de code-barres permettant d'entrer automatiquement des informations de code-barres, un dispositif (16) d'incubation par attraction magnétique et un dispositif (17) d'ajout automatique d'échantillon permettant de transférer un liquide dans une chambre (031) de réaction de la bandelette (03) réactive d'extraction ou un tube (021) PCR et de sceller le tube (021) PCR ;
le système de scellement et de transfert comprend un dispositif (21) de transfert PCR et un dispositif (22) de mélange à grande vitesse, et le dispositif (21) de transfert PCR est configuré pour saisir le tube (021) PCR et transférer le tube (021) PCR au dispositif (22) de mélange à grande vitesse pour un mélange à grande vitesse ; et
le système de détection d'acides nucléiques comprend un dispositif (31) d'amplification PCR et un dispositif (32) de détection par fluorescence.

2. Système de détection et de co-extraction d'acides nucléiques d'ADN et/ou d'ARN selon la revendication 1, dans lequel le panier (11) comprend une plaque (112) de couvercle de panier, une plaque (111) de fond de panier permettant de maintenir la bandelette (03) réactive d'extraction, et un porte-échantillon (113) permettant de maintenir le tube (04) d'échantillon, le porte-échantillon (113) étant relié de manière détachable à la plaque (111) de fond de panier ; et
la plaque (112) de couvercle de panier est reliée de manière rotative à la plaque (111) de fond de panier pour limiter un déplacement de la bandelette (03) réactive d'extraction dans une direction de hauteur lorsque la plaque (112) de couvercle de panier vient au contact de la plaque (111) de fond de panier.

3. Système de détection et de co-extraction d'acides nucléiques d'ADN et/ou d'ARN selon la revendication 1, dans lequel le dispositif (15) de balayage de code-barres comprend un support (151) de montage, un ensemble (153) de balayage, un ensemble (154) poussoir permettant de pousser la bandelette (03) réactive d'extraction entre une position de balayage et une position d'attente, un ensemble (152) de déplacement de poussoir permettant d'entraîner l'ensemble (154) poussoir pour qu'il se déplace le long d'une direction de longueur du support (151) de montage et un ensemble (155) de détection,
l'ensemble (155) de détection est configuré pour déterminer s'il y a une bandelette (03) réactive d'extraction et un tube (04) d'échantillon dans un canal courant, l'ensemble (155) de détection se trouve en liaison de signal avec l'ensemble (152) de déplacement de poussoir, et l'ensemble (153) de balayage et l'ensemble (155) de détection sont montés sur une plaque de base de montage de l'ensemble (154) poussoir.

4. Système de détection et de co-extraction d'acides nucléiques d'ADN et/ou d'ARN selon la revendication 1, dans lequel le dispositif (16) d'incubation par attraction magnétique comprend un module (161) d'incubation permettant de chauffer la chambre (031) de réaction, un module (162) d'attraction magnétique permettant d'attirer magnétiquement la chambre (031) de réaction, et un ensemble (163) d'entraînement, et, le module (161) d'incubation et le module (162) d'attraction magnétique sont tous deux reliés à l'ensemble (163) d'entraînement, l'ensemble (163) d'entraînement est configuré pour entraîner le module (161) d'incubation et le module (162) d'attraction magnétique afin qu'ils s'approchent alternativement de la chambre (031) de réaction.

5. Système de détection et de co-extraction d'acides nucléiques d'ADN et/ou d'ARN selon la revendication 4, dans lequel le module (161) d'incubation comprend un bloc (1611) chauffant présentant une rainure (1611-1) de réactif, un ensemble (1612) chauffant venant au contact d'une paroi intérieure de la rainure (1611-1) de réactif, un ensemble (1613) de dissipation thermique fourni au niveau d'un fond du bloc (1611) chauffant, et un capteur (1614) de température permettant de détecter la température du bloc (1611) chauffant, et, le capteur (1614) de température est relié au bloc (1611) chauffant.

6. Système de détection et de co-extraction d'acides nucléiques d'ADN et/ou d'ARN selon la revendication 4, dans lequel le module (162) d'attraction magnétique comprend un aimant (1621) et un ensemble (1622) rotatif permettant d'entraîner l'aimant (1621) pour qu'il tourne, l'ensemble (1622) rotatif étant relié à l'aimant (1621) ;
l'aimant (1621) comprend une première surface (1621-1) d'attraction magnétique et une deuxième surface (1621-2) d'attraction magnétique, et la première surface (1621-1) d'attraction magnétique et la deuxième surface (1621-2) d'attraction magnétique présentent des zones différentes et sont configurées pour attirer des bille s magnétiques dans la chambre (031) de réaction.

7. Système de détection et de co-extraction d'acides nucléiques d'ADN et/ou d'ARN selon la revendication 1, dans lequel le dispositif (21) de transfert PCR comprend un ensemble (211) préhenseur permettant de saisir ou de relâcher le tube (021) PCR, un ensemble (212) d'ouverture de couvercle permettant d'ouvrir ou de fermer le couvercle (314) d'amplification du dispositif (31) d'amplification PCR, un ensemble (213) d'axe X permettant d'entraîner l'ensemble (211) préhenseur et l'ensemble (212) d'ouverture de couvercle pour qu'ils se déplacent le long d'une direction d'axe X, un ensemble (214) d'axe Y permettant d'entraîner l'ensemble (211) préhenseur et l'ensemble (212) d'ouverture de couvercle pour qu'ils se déplacent le long d'une direction d'axe Y, et un ensemble (215) d'axe Z permettant d'entraîner l'ensemble (211) préhenseur et l'ensemble (212) d'ouverture de couvercle pour qu'ils se déplacent le long d'une direction d'axe Z ; et
l'ensemble (211) préhenseur et l'ensemble (212) d'ouverture de couvercle sont reliés de manière coulissante à l'ensemble (215) d'axe Z, l'ensemble (215) d'axe Z est relié de manière coulissante à l'ensemble (214) d'axe Y, et l'ensemble (214) d'axe Y est relié de manière coulissante à l'ensemble (213) d'axe X.

8. Système de détection et de co-extraction d'acides nucléiques d'ADN et/ou d'ARN selon la revendication 7, dans lequel l'ensemble (211) préhenseur comprend une plaque de base de préhenseur, une plaque mobile de préhenseur parallèle à un fond de la plaque de base de préhenseur, un préhenseur permettant de saisir le tube (021) PCR, et un ensemble d'entraînement de rétraction permettant de commander l'ouverture ou la rétraction du préhenseur, l'ensemble d'entraînement de rétraction est agencé sur la plaque de base de préhenseur, et une extrémité télescopique de l'ensemble d'entraînement de rétraction pénètre à travers la plaque de base de préhenseur pour être reliée à la plaque mobile de préhenseur, de manière à entraîner la plaque mobile de préhenseur afin qu'elle s'élève ou descende ; et
la plaque de fond de préhenseur et la plaque mobile de préhenseur sont reliées à l'ensemble d'entraînement de rétraction, le préhenseur s'ouvre lorsque la plaque mobile de préhenseur descend, et le préhenseur se rétracte lorsque la plaque mobile de préhenseur s'élève.

9. Système de détection et de co-extraction d'acides nucléiques d'ADN et/ou d'ARN selon la revendication 1, dans lequel le dispositif (31) d'amplification PCR comprend un bloc (311) chauffant d'amplification, une plaque (312) chauffante d'amplification, un radiateur (313) d'amplification, un couvercle (314) d'amplification permettant de recouvrir le bloc (311) chauffant d'amplification, et un ensemble (315) de refroidissement d'amplification permettant d'améliorer l'efficacité de dissipation thermique du radiateur (313) d'amplification, le bloc (311) chauffant d'amplification présente au moins deux orifices d'amplification permettant de placer les tubes (021) PCR, et, le couvercle (314) d'amplification, le bloc (311) chauffant d'amplification, la plaque (312) chauffante d'amplification, et le radiateur (313) d'amplification sont agencés séquentiellement de haut en bas.

10. Système de détection et de co-extraction d'acides nucléiques d'ADN et/ou d'ARN selon la revendication 9, dans lequel le dispositif (32) de détection par fluorescence comprend un ensemble (321) de source de lumière permettant de fournir une source de lumière fluorescente, un ensemble (322) de détection par fluorescence pour une détection quantitative, une fibre optique (323) de source de lumière, et une fibre optique (324) de détection par fluorescence, et, un côté de l'orifice d'amplification est relié à une extrémité de la fibre optique (323) de source de lumière, et l'autre côté de l'orifice d'amplification est relié à une extrémité de la fibre optique (324) de détection par fluorescence ; et
l'autre extrémité de la fibre optique (323) de source de lumière est reliée à l'ensemble (321) de source de lumière, et l'autre extrémité de la fibre optique (324) de détection par fluorescence est reliée à l'ensemble (322) de détection par fluorescence.

11. Système de détection et de co-extraction d'acides nucléiques d'ADN et/ou d'ARN selon l'une quelconque des revendications 1 à 10, dans lequel le dispositif (17) d'ajout automatique d'échantillon comprend un ensemble (171) aiguille de réactif pour l'extraction et l'injection d'un réactif, un ensemble (172) aiguille d'ajout d'échantillon pour l'extraction et l'injection d'une infusion d'échantillon et un ensemble (173) portique, l'ensemble (171) aiguille de réactif, l'ensemble (172) aiguille d'ajout d'échantillon et l'ensemble (173) portique étant reliés au système de commande ; et
l'ensemble (171) aiguille de réactif et l'ensemble (172) aiguille d'ajout d'échantillon sont respectivement agencés des deux côtés de l'ensemble (173) portique, et l'ensemble (173) portique est configuré pour se déplacer le long du panier (11), du module d'alimentation en consommables, et du module (14) de chargement de kit.

12. Système de détection et de co-extraction d'acides nucléiques d'ADN et/ou d'ARN selon la revendication 11, dans lequel l'ensemble (171) aiguille de réactif comprend une aiguille de réactif, un ensemble (1713) de commande de piquage, un ensemble de commande de pipetage, un ensemble de levage d'aiguille de réactif, et un ensemble (1714) de translation d'aiguille de réactif, et, l'aiguille de réactif comprend un adaptateur (1711) et une pompe (1712) à réactif communiquant avec l'adaptateur (1711), la pompe (1712) à réactif est reliée à l'ensemble de commande de pipetage, et l'adaptateur (1711) est relié à l'ensemble (1713) de commande de piquage pour que l'ensemble (1713) de commande de piquage commande l'adaptateur (1711) afin qu'il pique ou fasse sortir un embout (01) ou un bouchon (022) de scellement ; et
l'ensemble (1714) de translation d'aiguille de réactif est configuré pour entraîner l'aiguille de réactif afin qu'elle se déplace le long d'une direction perpendiculaire à une direction de mouvement de l'ensemble (173) portique dans un plan horizontal, et l'ensemble de levage d'aiguille de réactif est configuré pour entraîner l'aiguille de réactif afin qu'elle se déplace le long d'une direction verticale.

13. Système de détection et de co-extraction d'acides nucléiques d'ADN et/ou d'ARN selon la revendication 11, dans lequel l'ensemble (172) aiguille d'ajout d'échantillon comprend une pluralité de pompes (1721) à pipette fournies en parallèle, un ensemble (1723) de levage de pompes à pipette permettant d'entraîner la pluralité de pompes (1721) à pipette pour qu'elles montent et descendent, et un ensemble (1722) de levage de pistons permettant d'entraîner des pistons de la pluralité de pompes (1721) à pipette pour qu'ils coulissent par rapport à des corps de pompe de la pluralité de pompes (1721) à pipette,
la pluralité de pompes (1721) à pipette sont installées sur l'ensemble (1723) de levage de pompes à pipette, et l'ensemble (1722) de levage de pistons est relié aux pistons de la pluralité de pompes (1721) à pipette.
